# EUROPEAN PATENT APPLICATION

(11) **EP 2 390 666 A1**
(43) Date of publication of application: **30.11.2011**
(21) Application number: 11172878.8
(22) Date of filing: 21.03.2007
(51) Int. Cl.: G01N 33/574

(54) **N-Cadherin as target for cancer diagnosis and therapy**

(30) Priority: 21.03.2006 US 784734 P
(62) Divisional of application: 07753691.0
(71) Applicant: The Regents of The University of California, Oakland, CA 94607-5200 (US)
(72) Inventor: Reiter, Robert E., Los Angeles, CA California 90024-6017 (US)
(74) Representative: Müller, Christian Stefan Gerd

(57) **Abstract**

The present invention provides methods of diagnosis, providing a prognosis and a therapeutic target for the treatment of cancers that overexpress N-cadherin and Ly6-E, including prostate and bladder cancers. The invention further provides methods of drug discovery to identify pharmaceutical agents that inhibit or prevent the binding of N-cadherin and Ly6-E to its receptor, which are useful when used alone or in combination with known chemotherapeutics, immunotherapeutics, and radiotherapy for the reversal of resistance, tumor progression, and metastasis of cancers associated with the overexpession of N-cadherin and Ly6-E.

## Description

### BACKGROUND OF THE INVENTION

### INTRODUCTION

Prostate cancer is the most common malignancy and the second leading cause of cancer-related death in American men. Prostate cancer is a biologically and clinically heterogeneous disease. A majority of men with this malignancy harbor slow-growing tumors that may not impact an individual's natural lifespan, while others are struck by rapidly progressive, metastatic tumors. PSA screening is limited by a lack of specificity and an inability to predict which patients are at risk to develop hormone refractory metastatic disease. Recent studies advocating a lower PSA threshold for diagnosis may increase the number of prostate cancer diagnoses and further complicate the identification of patients with indolent vs. aggressive cancers (Punglia et al., N Engl J Med, 349: 335-342 (2003)). New serum and tissue markers that correlate with clinical outcome or identify patients with potentially aggressive disease are urgently needed (Welsh et al., Proc Natl Acad Sci U S A, 100: 3410-3415 (2003)).

Recent expression profiling studies suggest that expression signatures for metastatic vs. non-metastatic tumors may reside in the primary tumor (Ramaswamy et al., Nat Genet, 33: 49-54 (2003); Sotiriou et al., Proc Natl Acad Sci USA, 100: 10393-10398 (2003)). Additional features that predispose tumors to metastasize to specific organs may also be present at some frequency in the primary tumor (Kang et al., Cancer Cell, 3: 537-549 (2003)). These recent observations suggest that novel markers of pre-metastatic or pre-hormone refractory prostate cancer may be identified in early stage disease. These markers may also play a role in the biology of metastatic or hormone refractory prostate cancer progression. Recent examples of genes present in primary tumors that correlate with outcome and play a role in the biology of prostate cancer progression include EZH2 and LIM kinase (Varambally et al., Nature, 419: 624-629 (2002); Yoshioka et al., Proc Natl Acad Sci USA, 100: 7247-7252 (2003)). However, neither of these two genes is secreted.

In order to identify new candidate serum or tissue markers of hormone refractory prostate cancer, we compared gene expression profiles of paired hormone dependent and hormone refractory prostate cancer xenografts. The LAPC-9 xenograft was established from an osteoblastic bone metastasis and progresses from androgen dependence to independence following castration in immune deficient mice (Craft et al., Cancer Research, In Press (1999)). It has been used previously to identify candidate therapeutic targets in prostate cancer. Differentially expressed genes were validated and then examined for sequence homology to secreted or cell surface proteins. We report here on the identification, characterization and initial validation of two such candidate genes, Ly6 E and N-Cadherin, which are overexpressed in both hormone refractory prostate cancer and bladder cancer.

Accordingly, the invention provides compositions and methods which target N-Cadherin or LY6-E in the diagnosis, prognosis, and treatment of cancers overexpressing Ly6 E and/or N-Cadherin including, but not limited to, prostate cancer and bladder cancer.

### BRIEF SUMMARY OF THE INVENTION

In a first aspect, the present invention provides methods of diagnosis and prognosis for individuals at risk for cancers that overexpress a N-Cadherin or LY6-E protein or mRNA transcript, particularly prostate and/or bladder cancers. The methods generally comprise obtaining a test tissue sample from an individual at risk of having a cancer that overexpresses a N-Cadherin or LY6-E protein or mRNA transcript and determining the presence or absence or amount ofN-Cadherin or LY6-E protein or mRNA transcript in the test tissue sample in comparison to a control tissue sample from an individual known to be negative for cancer. Typically, the tissue sample is serum, but can also be biopsy tissue, including prostate tissue or bladder tissue.

Further, N-Cadherin or LY6-E represent useful prostate and bladder cancer markers for discriminating between malignant or invasive prostate and bladder cancers, normal prostate glands and bladder tissues and noin-malignant neoplasias of the prostrate and bladder. For example, N-Cadherin or LY6-E is over expressed in prostate cancer in relation to benign prostatic hyperplasia (BPH). These markers can help in the prognosis of whether a cancer (e.g., bladder cancer, prostate cancer) will progress to a treatment resistant or hormone independent state, become invasive, and/or metastasize. In some embodiments of the above, E-cadherin levels are also used in the prognosis as underexpression of E-cadherin is associated with more aggressive cancers which are likely to be invasive and to metastasize. levels are underexpressed. Accordingly, in some embodiments, both E-cadherin and N-cadherin levels are determined.

The present invention further provides methods of inhibiting the growth of and promoting the regression of a cancerous tumor that overexpresses a N-Cadherin or LY6-E protein or mRNA transcript, the method comprising inhibiting or reducing the binding of N-Cadherin or LY6-E protein to a N-Cadherin or LY6-E receptor, respectively, on a cell of the tumor tissue. The methods find particular use in treating any cancer that overexpresses a N-Cadherin or LY6-E protein or mRNA transcript, including prostate and bladder cancers.

The present invention also provides methods of identifying compounds that inhibit the binding of a N-Cadherin or LY6-E protein, respectively, to a N-Cadherin or LY6-E receptor, respectively, wherein said compounds find use in inhibiting the growth of and promoting the regression of a cancerous tumor that overexpresses N-Cadherin or LY6-E protein, respectively, for example, a tumor of a urogenital tissue including a prostate or bladder cancer tumor. The screening methods can be carried out in *vitro* (*i.e.*, by ELISA) and *in vivo.*

In some embodiments, the invention provides methods of diagnosing a cancer in a subject by determining the level of N-Cadherin or LY6-E protein expression or activity in a biological sample or biopsy of the cancer or tumor from the subject wherein an increased level of N-Cadherin or LY6-E protein expression or activity in the sample or biopsy is indicative of cancer. In some embodiments, determining the N-Cadherin or LY6-E protein levels involves steps of (a) contacting a tissue sample or biopsy from the subject with an antibody that specifically binds to N-Cadherin or LY6-E protein; and (b) determining whether or not N-Cadherin or LY6-E protein is overexpressed in the sample or biopsy; thereby diagnosing the cancer. In a further embodiment of such, the cancer can be a prostate cancer, ovarian cancer, renal cancer, breast cancer, colon cancer, lung cancer, leukemia, non-Hodgkin's lymphoma, multiple myeloma, or hepatocarcinoma. In some further embodiments, still the tissue sample can be a needle biopsy, a surgical biopsy or a bone marrow biopsy. A tissue sample can be fixed or embedded in paraffin. A tissue sample can be, for instance, from prostate, ovary, bone, blood, lymph node, liver, or kidney. The antibody in some embodiments is a monoclonal antibody. An elevated level of N-Cadherin or LY6-E protein in a sample is indicative of cancer. In a preferred embodiment, the cancer is a prostate cancer or bladder cancer. In preferred embodiments, the diagnosis of cancer is made upon the basis of the N-Cadherin or LY6-E protein levels as well as on other conventional indicators of cancer. For instance, the diagnosis can be based upon both the N-Cadherin or LY6-E protein findings and the histology or growth characteristics of the cancer cells. In the case of prostate cancer, for instance, the N-Cadherin or LY6-E protein findings can supplement the Gleason scoring system to provide a more accurate or reliable indicator of carcinogenicity and likelihood of disease progression. In some embodiments of the above, the diagnosis is also based upon E-cadherin levels which can be similarly determined.

In other embodiments of any of the above, the method alternatively determines the N-Cadherin or LY6-E protein level by (a) contacting a tissue sample with a primer set of a first oligonucleotide and a second oligonucleotide that each specifically hybridize to N-Cadherin or LY6-E protein 3 nucleic acid; (b) amplifying N-Cadherin or LY6-E protein nucleic acid in the sample; and (c) determining whether or not N-Cadherin or LY6-E protein nucleic acid is overexpressed in the sample; thereby diagnosing the cancer. The first oligonucleotide can comprise a nucleotide sequence of N-Cadherin or LY6-E cDNA and the second oligonucleotide can comprise a nucleotide sequence complementary to that of N-Cadherin or LY6-E 3 cDNA. Preferably, both nucleotides are less than 50 base pairs in length. In a preferred embodiment, the cancer is a prostate or bladder cancer. In some embodiments of the above, the diagnosis is also based upon E-cadherin levels which can be similarly determined.

In some aspects, the invention provides a method of prognosis for a cancer that overexpresses N-Cadherin or LY6-E by assessing the likelihood that the cancer will be invasive, metastasize, recur or be resistant to therapy. In a first embodiment in this aspect, the invention provides a method of further diagnosing a cancer that overexpresses N-Cadherin or LY6-E or has increased N-Cadherin or LY6-E transcriptional activity and therefore has an increased liklihood of invasiveness, metastasizing, recurrence or resistance to therapy. The method comprises the steps of (a) contacting a tissue sample with an antibody that specifically binds to N-Cadherin or LY6-E; and (b) determining whether or not the N-Cadherin or LY6-E is overexpressed in the sample; thereby diagnosing the cancer that overexpresses N-Cadherin or LY6-E. The cancer may be diagnosed before or after obtaining and analyzing the sample for N-Cadherin or LY6-E expression or activity levels. The cancer may have been identified on the basis of histological appearance (e.g., Gleason score in the case of prostate cancer) and not on the basis of the N-Cadherin or LY6-E level determination. The cancer can have been diagnosed as such with or without, or despite, knowledge of an elevated N-Cadherin or LY6-E level. In a further embodiment of such, the cancer can be a prostate cancer or bladder cancer, renal cancer, breast cancer, colon cancer, lung cancer, leukemia, non-Hodgkin's lymphoma, multiple myeloma, or hepatocarcinoma. In some further embodiments, still the tissue sample can be a needle biopsy, a surgical biopsy or a bone marrow biopsy. A tissue sample can be fixed or embedded in paraffin. A tissue sample can be, for instance, from prostate, ovary, bone, lymph node, liver, or kidney. The antibody in some embodiments is a monoclonal antibody. An elevated level of N-Cadherin or LY6-E in a sample is prognostic of, and associated with, an increased risk of recurrence or resistance to therapy for the cancer. In a preferred embodiment, the cancer is a prostate cancer or bladder cancer. In some embodiments of the above, the diagnosis is also based upon E-cadherin levels which can be similarly determined.

In other embodiments of the above for this second aspect, the method of diagnosing a cancer that overexpresses N-Cadherin or LY6-E comprises the steps of (a) contacting a tissue sample with a primer set of a first oligonucleotide and a second oligonucleotide that each specifically hybridize to N-Cadherin or LY6-E nucleic acid; (b) amplifying N-Cadherin or LY6-E nucleic acid in the sample; and (c) determining whether or not N-Cadherin or LY6-E nucleic acid is overexpressed in the sample; thereby diagnosing the cancer that overexpresses N-Cadherin or LY6-E. The first oligonucleotide can comprise a nucleotide sequence of N-Cadherin or LY6-E cDNA and the second oligonucleotide can comprise a nucleotide sequence complementary to that of N-Cadherin or LY6-E cDNA. Preferably, both nucleotides are less than 50 base pairs in length. In the above methods, a increased level of N-Cadherin or LY6-E in a sample is, prognostic for, and associated with, an increased risk of recurrence, metastasis, hormone independende, or resistance to therapy for the cancer. In a preferred embodiment, the cancer is a prostate or bladder cancer. In some embodiments of the above, the diagnosis is also based upon E-cadherin levels which can be similarly determined.

In yet other embodiments, the invention provides a method of targeting patients for more aggressive or alternative cancer therapy or increased surveillance for a cancer recurrence based upon an elevated level of N-Cadherin or LY6-E in a tissue sample from the patient taken before, during, or after surgical removal of the cancerous tissue (e.g., prostectomy) or before, during, or after another cancer treatment. The N-Cadherin or LY6-E activity or expression levels can be determined as described above. In some embodiments of the above, the diagnosis is also based upon E-cadherin levels which can be similarly determined. The cancer that overexpresses N-Cadherin or LY6-E can be, for instance, a prostate cancer, ovarian cancer, renal cancer, lung cancer, breast cancer, colon cancer, leukemia, non-Hodgkin's lymphoma, multiple myeloma or hepatocarcinoma. In a preferred embodiment, the cancer is a prostate or bladder cancer. Patients identified as having raised N-Cadherin or LY6-E levels and accordingly being at high risk of metastasis, recurrence or a therapy resistant cancer can be treated with exogenous or endogenous hormone ablation, optionally supplemented with chemotherapy and/or radiation. In the case of prostate cancer, the hormone ablation is androgen ablation (e.g., treatment with finasteride and other anti-tesosterone or anti-DHT agents).

In some embodiments, the invention provides a method of treating or inhibiting a cancer, a therapy resistant cancer, a metastasis of cancer, or recurrence of cancer, that overrexpresses N-Cadherin or LY6-E in a subject comprising administering to the subject a therapeutically effective amount of one or more inhibitors of N-Cadherin or LY6-E or N-Cadherin or LY6-E expression. The cancer that overexpresses N-Cadherin or LY6-E can be, for instance, a prostate cancer, bladder cancer, ovarian cancer, renal cancer, lung cancer, breast cancer, colon cancer, leukemia, non-Hodgkin's lymphoma, multiple myeloma or hepatocarcinoma. In a preferred embodiment, the cancer is a prostate or bladder cancer. The compound can be a compound as identified in the following aspect. The overexpression can be identified as described in the previous aspects. The compound can be administered concurrently with another cancer therapy.

The invention also provides a method of identifying a compound that inhibits cancer, therapy resistant cancer, or metastasis, or a recurrence of cancer, the method comprising the steps of contacting a cell with a compound; and determining the effect of the compound on the expression or activity of the N-Cadherin or LY6-E polypeptide in the cell; wherein compounds which decrease the N-Cadherin or LY6-E expression or activity levels are identified as being able to inhibit cancer, its metastasis, or progression to a hormone-independent or treatment resistant state. In some embodiments, the compound decreases the expression of N-Gadherin or LY6-E in the cell. In yet other embodiments, the cell is a cancer cell and, more particularly, may be cancer cell of a particular tissue type or origin (e.g., prostate, ovary, kidney, lung, breast, colon, leukemia, non-Hodgkin's lymphoma, multiple myeloma or hepatocarcinoma) which has overexpression of N-Cadherin or LY6-E. In still further embodiments, the cancer that overexpresses N-Cadherin or LY6-E is prostate cancer, bladder cancer, ovarian cancer, renal cancer, lung cancer, breast cancer, colon cancer, leukemia, non-Hodgkin's lymphoma, multiple myeloma or hepatocarcinoma. In a preferred embodiment, the cancer is a prostate cancer or bladder cancer.

The invention also provides a method of localizing a cancer that overexpresses N-Cadherin or LY6-E *in vivo,* and is therefore likely to be invasive, likely to metastasize, become hormone independent, or refractory to treatment, the method comprising the step of imaging in a subject a cell overexpressing N-Cadherin or LY6-E wherein the cancer that overexpresses N-Cadherin or LY6-E is selected from the group consisting of prostate cancer, bladder cancer, ovarian cancer, renal cancer, breast cancer, lung cancer, colon cancer, leukemia, non-Hodgkin's lymphoma, multiple myeloma and hepatocarcinoma.

In addition, N-Cadherin or LY6-E proteins and N-Cadherin or LY6-E -encoding nucleic acid molecules may be used in various immunotherapeutic methods to promote immune-mediated destruction of cancers (e.g., prostate or bladder tumors), particularly, when such tumors are invasive.

In some embodiments, the invention provides methods of treating cancer, particularly an invasive cancer or a metastasis, or preventing the progression of a cancer to a treatment resistant, hormone-indepenent, or metastasizing state by administering antibodies that bind to N-Cadherin and LY6-E to reduce their respective activity in the patient. Additionally, in some other embodiments, the antibodies are conjugated to effector moieties which thereby are preferentially cytotoxic to cells overexpressing the N-Cadherin or LY6-E. In some embodiments, the antibodies are humanized monoclonal antibodies.

In some embodiments, the invention provides methods of treating cancer or preventing the progression of a cancer to a treatment resistant, hormone-independent, or metastasizing state by administration of RNAi molecule or an antisense molecule specific for N-cadherin or LY6-E and which accordingly are capable of inhibit the expression ofN-Cadherin or LY6-E. In some embodiments, the RNAi molecule may be a short hairpin RNAi.

In another aspect, the invention provides antibodies to N-cadherin or Ly6-E. N-cadherin or Ly6-E antibodies may be used in diagnosis, prognosis, or the treatment of a cancer (e.g., prostate or bladder cancer) alone or when conjugated with an effector moiety. N-cadherin or Ly6-E antibodies conjugated with toxic agents, such as ricin, as well as unconjugated antibodies may be useful therapeutic agents naturally targeted to N-cadherin or Ly6-E -bearing prostate cancer cells. Such antibodies can be useful in blocking invasiveness. Suitable N-cadherin antibodies for use according to the invention include, but are not limited to, GC4, 1H7, 1F12, 2B3.

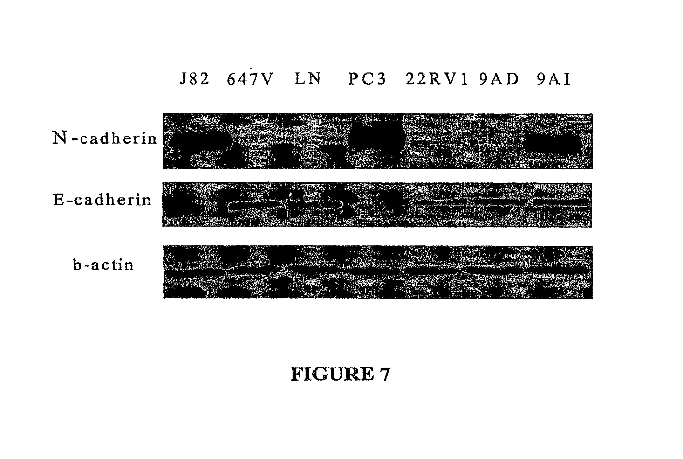

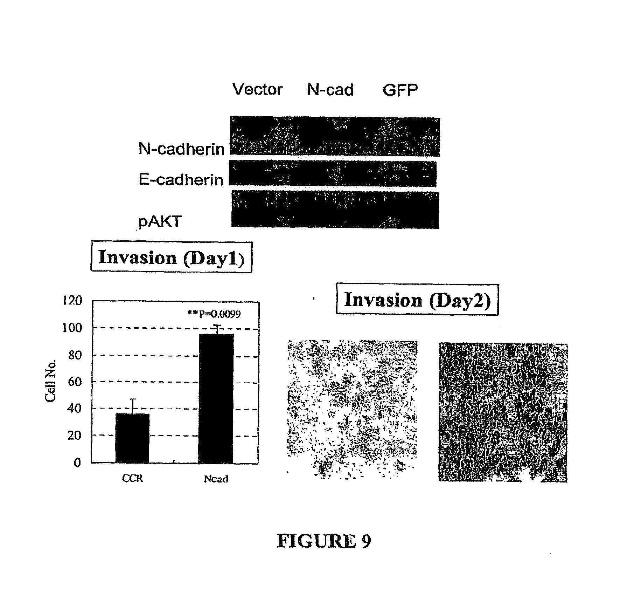

In any of the above aspects and embodiments, the tissue, cancer, subject, or patient to be treated is human or mammalian. In any of the above aspects and embodiments, the cancer can be an androgen independent cancer.
In particular, the invention pertains to the following:
1. A method of diagnosing a urogenital cancer that overexpresses a N-cadherin protein, the method comprising the steps of :
   (a) obtaining a test tissue sample from an individual at risk of having a cancer that overexpresses a N-cadherin protein; and
   (b) determining the presence or absence or amount of the N-cadherin protein in the test tissue sample in comparison to a control tissue sample from an individual known to be negative for the cancer; thereby diagnosing said cancer that overexpresses a N-cadherin protein.
2. The method of item 1, wherein the test tissue is contacted with an antibody that specifically binds to a N-cadherin protein, whereby the overexpression of the N-cadherin protein is determined.
3. The method of item 1, wherein N-cadherin inRNA is also overexpressed and the test tissue sample is contacted with a primer set of a first oligonucleotide and a second oligonucleotide that each specifically hybridize to the N-cadherin mRNA nucleic acid to amplify the N-cadherin mRNA nucleic acid; whereby the overexpression of the N-cadherin protein is also determined.
4. The method of item 1, wherein said tissue sample is a serum or a blood sample.
5. The method of item 1, wherein said tissue sample is prostate or bladder tissue.
6. The method of item 1, wherein said cancer is a prostate cancer.
7. The method of item 6, wherein said cancer is a bladder cancer.
8. The method of item 1, wherein said cancer is a hormone refractory prostate cancer.
9. The method of item 1, wherein said cancer is a metastatic cancer.
10. The method of item 2, wherein said antibody is a monoclonal antibody.
11. The method of item 1, wherein the overexpression is by at least four-fold greater than levels in the control sample.
12. The method of item 3, wherein the determining of the presence or absence or amount of the mRNA transcript is by PCR.
13. The method of item 1, wherein further the presence or absence or amount of the E-cadherin in the test tissue sample is determined in comparison to a control tissue sample from an individual known to be negative for cancer;
   wherein underexpression of E-cadherin is further indicative that the cancer is likely to become invasive, metastasize, become hormone independent, or refractory to treatment.
14. A method of providing a cancer prognosis, the method comprising the steps of:
   (a) contacting a test tissue sample from an individual at risk of having the cancer; and
   (b) determining the presence or absence or amount of the N-cadherin protein in the test tissue sample in comparison to a control tissue sample from an individual known to be negative for the cancer; thereby identifying the cancer as overexpressing a N-cadherin mRNA transcript.
15. The method of item 14, wherein the test tissue sample is contacted with a primer set of a first oligonucleotide and a second oligonucleotide that each specifically hybridize to a N-cadherin mRNA nucleic acid to amplify the N-cadherin mRNA nucleic acid in the sample; whereby the presence or absence or the amount of the N-cadherin protein is determined.
16. The method of item 14, wherein the test tissue sample is contacted with with an antibody that specifically binds to the N-cadherinprotein whereby the comparative presence or absence or the amount of the N-cadherin protein is determined.
17. The method of item 14, wherein the cancer is a urogenital cancer.
18. The method of item 14, wherein the cancer is prostate cancer.
19. The method of item 14, wherein the cancer is bladder cancer.
20. The method of item 14, wherein an overexpression of N-cadherin indicates that the cancer is likely to become invasive, metastasize, become hormone independent, or become refractory treatment.
21. The method of item 20, wherein the overexpression is by at least four-fold over the control sample.
22. The method of item 14, wherein further the presence or absence or amount of the E-cadherin in the test tissue sample is determined in comparison to a control tissue sample from an individual known to be negative for cancer;
   wherein underexpression of E-cadherin is further indicative that the cancer is likely to become invasive, metastasize, become hormone independent, or refractory to treatment.
23. A method of identifying a compound that inhibits a cancer associated with the overexpression of N-cadherin, the method comprising the steps of:
   (a) contacting a cell expressing N-cadherin protein and a N-cadherin receptor with a compound; and
   (b) determining whether said compound inhibits the binding of N-cadherin protein to the N-cadherin receptor; thereby identifying a compound that inhibits a cancer associated with the overexpression of N-cadherin.
24. The method of item 23, wherein said cancer is a urogenital cancer.
25. The method of item 24, wherein said cancer is a prostate cancer or a bladder cancer.
26. A method of treating cancer overexpressing N-cadherin, the method comprising administering a therapeutically effective amount of a compound that inhibits the binding ofN-cadherin protein to a N-cadherin receptor in a prostate tissue cell.
27. The method of item 26, wherein said cancer is a urogenital cancer.
28. The method of item 27, wherein said cancer is a prostate cancer or a bladder cancer.
29. A method of treating cancer, said method comprising administration of of a therapeutically effective amount of an antibody which binds to N-cadherin.
30. The method of item 29, wherein the method inhibits the invasiveness or metastasis of the cancer.
31. The method of item 29, wherein the cancer is prostate or bladder cancer.
32. The method of any of items 29 to 31, wherein the cancer overexpresses N-cadherin.
33. A method of treating cancer, said method comprising administration of a therapeutically effective amount of an antibody which binds to N-cadherin, wherein the antibody is conjugated to an effector moiety.
34. The method of item 33, wherein the method inhibits the invasiveness or metastasis of the cancer.
35. The method of item 33, wherein the cancer is prostate or bladder cancer.
36. The method of any of items 33 to 35, wherein the cancer overexpresses N-cadherin.
37. The method of item 33, wherein the effector molecule is a cytotoxic agent.
38. The method of item 37, wherein the cytotoxic agent is selected from the group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria officinalis inhibitor, maytansinoids, and glucocorticoidricin
39. A method of treating cancer, said method comprising administration of of an siRNA which is capable of inhibiting or silencing the expression ofN-cadherin.
40. The method of item 39, wherein the expression of N-cadherin is inhibited and the RNAi has a sequence which is identical to a nucleic acid sequence of Figure 7.
41. The method of item 39, wherein the cancer is prostate or bladder cancer.
42. The method of any of items 39 to 41, wherein the cancer overexpresses N-cadherin.
43. The method of any one of items 39 to 41, wherein the siRNA is short hairpin RNA.
44. The method of any one of items 39 to 43, wherein the method inhibits the invasiveness or metastasis of the cancer.
45. A method of treating a cancer patient, comprising determining whether a cancer is likely to become invasive, metastasize, hormone independent, or refractory treatment according to the method of item 14, and administering a chemotherapeutic agent, an immunotherapeutic agent, hormonal therapy, or radiotherapy according to whether there is an increased likelihood of the cancer becoming invasive, metastasizing, hormone independent, or refractory to treatment.
46. The method of item 45, wherein the cancer is a urogenital cancer.
47. The method of item 45, wherein the cancer is prostate cancer.
48. The method of item 45, wherein the chemotherapeutic agent is selected from the group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria officinalis inhibitor, maytansinoids, and glucocorticoidricin.
49. The method of item 47, wherein the patient is treated by radical prostatectomy, radiation therapy, hormone therapy, or chemotherapy.
50. The method of items 45 to 49, wherein the overexpression ofN-cadherin is by at least four-fold over the control sample.
51. The method of item 45, wherein a N-cadherin inhibitor, N-cadherin siRNA, or anti-N-cadherin antibody is also administered.
52. A use of N-cadherin protein or mRNA as a target in the diagnosis, prognosis, or treatment of cancer.
53. The use of item 52, wherein the cancer is prostate cancer or bladder cancer.
54. The use of item 52, wherein the cancer overexpresses N-cadherin.
55. The use of item 53, wherein the cancer overexpresses N-cadherin by at least four-fold.
56. The use of item 52, wherein the N-cadherin protein is contacted with an anti-N-cadherin antibody.
57. The use of item 52, wherein siRNA which is capable of interfering with the expression of the mRNA is administered to a subject having a cancer which overexpresses N-cadherin.
58. The use of item 52 or 51, wherein the cancer is invasive or refractory to treatment.
59. A method of diagnosing a cancer that overexpresses a Ly6-E protein, the method comprising the steps of:
   (a) obtaining a test tissue sample from an individual at risk of having a cancer that overexpresses a Ly6-E protein; and
   (b) determining the presence or absence or amount of the Ly6-E protein in the test tissue sample in comparison to a control tissue sample from an individual known to be negative for the cancer; thereby diagnosing said cancer that overexpresses a Ly6-E protein.
60. The method of item 59, wherein the test tissue is contacted with an antibody that specifically binds to a Ly6-E protein, whereby the overexpression of the Ly6-E protein is determined.
61. The method of item 59, wherein Ly6-E mRNA is also overexpressed and the test tissue sample is contacted with a primer set of a first oligonucleotide and a second oligonucleotide that each specifically hybridize to the Ly6-E mRNA nucleic acid to amplify the Ly6-E mRNA nucleic acid; whereby the overexpression of the Ly6-E protein is also determined.
62. The method of item 59, wherein said tissue sample is a serum or a blood sample.
63. The method of item 59, wherein said tissue sample is prostate or bladder tissue.
64. The method of item 59, wherein said cancer is a prostate cancer.
65. The method of item 59, wherein said cancer is a bladder cancer.
66. The method of item 59, wherein said cancer is a hormone refractory prostate cancer.
67. The method of item 59, wherein said cancer is a metastatic cancer.
68. The method of item 60, wherein said antibody is a monoclonal antibody.
69. The method of item 59, wherein the overexpression is by at least four-fold greater than levels in the control sample.
70. The method of item 61, wherein the determining of the presence or absence or amount of the mRNA transcript is by PCR.
71. The method of item 59, wherein further the presence or absence or amount of the E-cadherin in the test tissue sample is determined in comparison to a control tissue sample from an individual known to be negative for cancer;
   wherein underexpression of E-cadherin is further indicative that the cancer is likely to become invasive, metastasize, become hormone independent, or refractory to treatment.
72. A method of providing a cancer prognosis, the method comprising the steps of :
   (a) contacting a test tissue sample from an individual at risk of having the cancer; and
   (b) determining the presence or absence or amount of the Ly6-E protein in the test tissue sample in comparison to a control tissue sample from an individual known to be negative for the cancer; thereby identifying the cancer as overexpressing a Ly6-E mRNA transcript.
73. The method of item 72, wherein the test tissue sample is contacted with a primer set of a first oligonucleotide and a second oligonucleotide that each specifically hybridize to a Ly6-E mRNA nucleic acid to amplify the Ly6-E mRNA nucleic acid in the sample; whereby the presence or absence or the amount of the Ly6-E protein is determined.
74. The method of item 72, wherein the test tissue sample is contacted with with an antibody that specifically binds to the Ly6-Eprotein whereby the comparative presence or absence or the amount of the Ly6-E protein is determined.
75. The method of item 72, wherein the cancer is a urogenital cancer.
76. The method of item 72, wherein the cancer is prostate cancer.
77. The method of item 72, wherein the cancer is bladder cancer.
78. The method of item 72, wherein an overexpression of Ly6-E indicates that the cancer is likely to become invasive, metastasize, become hormone independent, or become refractory treatment.
79. The method of item 78, wherein the overexpression is by at least four-fold over the control sample.
80. The method of item 72, wherein further the presence or absence or amount of the E-cadherin in the test tissue sample is determined in comparison to a control tissue sample from an individual known to be negative for cancer;
   wherein underexpression of E-cadherin is further indicative that the cancer is likely to become invasive, metastasize, become hormone independent, or refractory to treatment.
81. A method of identifying a compound that inhibits a cancer associated with the overexpression of Ly6-E, the method comprising the steps of:
   (a) contacting a cell expressing Ly6-E protein and a Ly6-E receptor with a compound; and
   (b) determining whether said compound inhibits the binding of Ly6-E protein to the Ly6-E receptor; thereby identifying a compound that inhibits a cancer associated with the overexpression of Ly6-E.
82. The method of item 81, wherein said cancer is a urogenital cancer.
83. The method of item 82, wherein said cancer is a prostate cancer or a bladder cancer.
84. A method of treating cancer overexpressing Ly6-E, the method comprising administering a therapeutically effective amount of a compound that inhibits the binding of Ly6-E protein to a Ly6-E receptor in a prostate tissue cell.
85. The method of item 84, wherein said cancer is a urogenital cancer.
86. The method of item 85, wherein said cancer is a prostate cancer or a bladder cancer.
87. A method of treating cancer, said method comprising administration of of a therapeutically effective amount of an antibody which binds to Ly6-E.
88. The method of item 87, wherein the method inhibits the invasiveness or metastasis of the cancer.
89. The method of item 87, wherein the cancer is prostate or bladder cancer.
90. The method of any of items 87 to 89, wherein the cancer overexpresses Ly6-E.
91. A method of treating cancer, said method comprising administration of a therapeutically effective amount of an antibody which binds to Ly6-E, wherein the antibody is conjugated to an effector moiety.
92. The method of item 91, wherein the method inhibits the invasiveness or metastasis of the cancer.
93. The method of item 91, wherein the cancer is prostate or bladder cancer.
94. The method of any of items 91 to 93, wherein the cancer overexpresses Ly6-E.
95. The method of item 91, wherein the effector molecule is a cytotoxic agent.
96. The method of 95, wherein the cytotoxic agent is selected from the group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria officinalis inhibitor, maytansinoids, and glucocorticoidricin
97. A method of treating cancer, said method comprising administration of of an siRNA which is capable of inhibiting or silencing the expression of Ly6-E.
98. The method of item 97, wherein the expression of Ly6-E is inhibited and the RNAi has a sequence which is identical to a nucleic acid sequence of Figure 9.
99. The method of item 98, wherein the cancer is prostate or bladder cancer.
100. The method of any of items 97 to 99, wherein the cancer overexpresses Ly6-E.
101. The method of any one of items 97 to 99, wherein the siRNA is short hairpin RNA.
102. The method of any one of items 97 to 99, wherein the method inhibits the invasiveness or metastasis of the cancer.
103. A method of treating a cancer patient, comprising determining whether a cancer is likely to become invasive, metastasize, hormone independent, or refractory treatment according to the method of item 14, and administering a chemotherapeutic agent, an immunotherapeutic agent, hormonal therapy, or radiotherapy according to whether there is an increased likelihood of the cancer becoming invasive, metastasizing, hormone independent, or refractory to treatment.
104. The method of item 103, wherein the cancer is a urogenital cancer.
105. The method of item 103, wherein the cancer is prostate cancer.
106. The method of item 103, wherein the chemotherapeutic agent is selected from the group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria officinalis inhibitor, maytansinoids, and glucocorticoidricin.
107. The method of item 103, wherein the patient is treated by radical prostatectomy, radiation therapy, hormone therapy, or chemotherapy.
108. The method of any one of items 103 to 107 wherein the overexpression of Ly6-E is by at least four-fold over the control sample.
109. The method of any one of items 103 to 107, wherein a Ly6-E inhibitor, Ly6-E siRNA, or anti-Ly6-E antibody is also administered.
110. A use of Ly6-E protein or mRNA as a target in the diagnosis, prognosis, or treatment of cancer.
111. The use of item 110, wherein the cancer is prostate cancer or bladder cancer.
112. The use of item 110, wherein the cancer overexpresses Ly6-E.
113. The use of item 110, wherein the cancer overexpresses Ly6-E by at least four-fold.
114. The use of item 110, wherein the Ly6-E protein is contacted with an anti-Ly6-E antibody.
115. The use of item 110, wherein siRNA which is capable of interfering with the expression of the mRNA is administered to a subject having a cancer which overexpresses Ly6-E.
116. The use of item 110, wherein the cancer is invasive or refractory to treatment.

### BRIEF DESCRIPTION OF THE DRAWINGS

**Figure 1****.** Endogenous Cadherin profiles in human bladder cancer cell lines. A. Western blot demonstrates that N-Cadherin protein levels directly correlates with pAkt levels and inversely correlates with E-Cadherin expression. B. PTEN status of human bladder cancer cell lines.

**Figure 2****.** Matrigel in-vitro invasion assay of human bladder cancer cell lines with varying cadherin profiles. Cell lines expressing E-Cadherin were less invasive than those expressing endogenous N-Cadherin. Experiments were performed in triplicate and averaged.

**Figure 3****.** Effects of N-Cadherin antibody neutralization on invasion. A. Human bladder cancer cell lines comparing treatment with the GC4 neutralizing antibody (white) vs. non-treated (black). B. Western blot analysis demonstrating that antibody neutralization results in decreased pAKT expression and increased E-cadherin expression in the endogenously N-Cadherin-expressing T24 cell line. The invasion assay data represents experiments performed in triplicates and averaged.

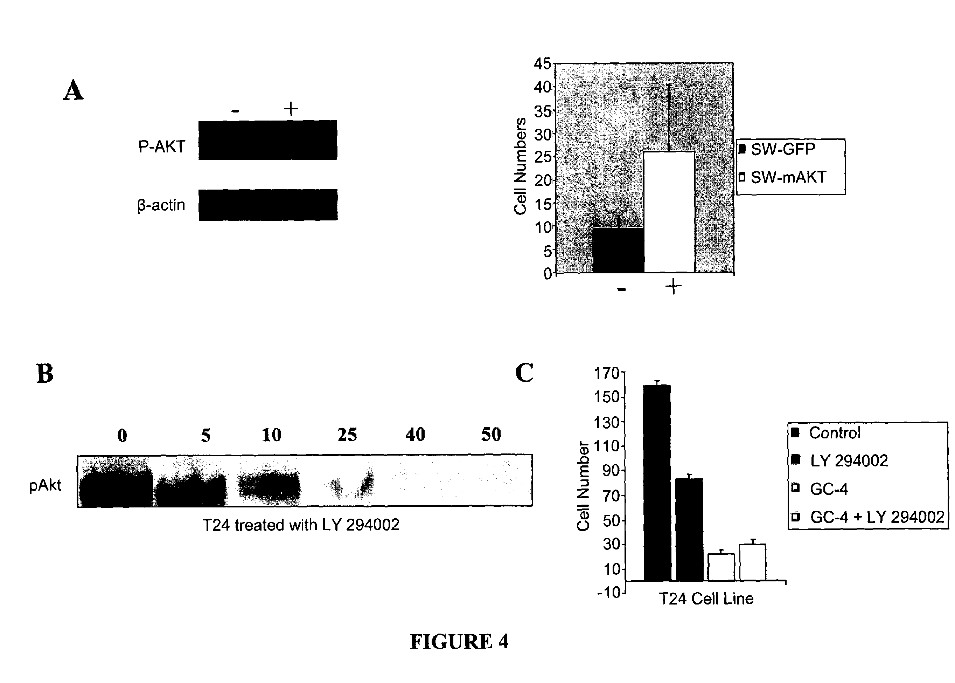

**Figure 4****.** The PI3/Akt pathway contributes to some, but not all of the invasive activity found in endogenously expressing N-cadherin bladder cancer cell lines. A. Forced expression of pAkt via lentiviral infection in N-Cadherin positive SW780 cells. No change in E-Cadherin expression is noted. B. The SW780 cell line with forced expression of pAkt shows increased invasive activity. C. The N-Cadherin positive cell line T24 was treated with the Akt inhibitor LY294002. Treatment with this inhibitor resulted in decreased pAkt expression. D. T24 cells treated with N-Cadherin neutralizing antibody resulted in decreased invasive potential when compared with Akt inhibitors alone.

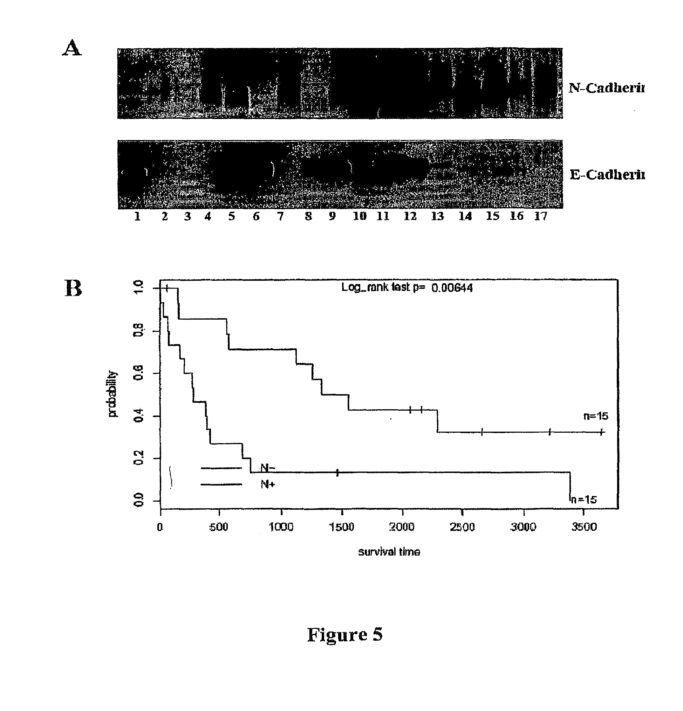

**Figure 5****.** N-Cadherin status correlates with decreased survival in patients with superficial and invasive bladder cancer. A. Western blot showing cadherin profiles of superficial and invasive human bladder tumors. Fourteen of seventeen patients expressed N-Cadherin. B. Kaplan-Meier curve of overall survival of patients grouped according to N-Cadherin status following radical cystectomy for invasive bladder cancer. N-Cadherin expression was determined from data collected on Affymetrix chips and based on RNA expression. Statistical analysis demonstrated that patients who were N-Cadherin positive (shown in blue) had decreased survival compared to N-Cadherin negative patients (shown in red).

**Figure 6****.** Cadherin status correlates with prognosis among patients with invasive bladder cancer. Patients were stratified based on both N-Cadherin and E-Cadherin and were divided into three groups. A Kaplan-Meier curve showing patients with N-Cadherin positive and E-Cadherin negative tumors had the worst overall survival. Those with E-Cadherin positive, N-Cadherin negative tumors fared the best while those with mixed profiles were intermediate.

**Figure 7****: Western blot analyses.** These analyses show N-Cadherin and E-Cadherin expression in bladder cancer cell lines (J82 and 647V) and prostate cancer cell lines (LNCaP, 22RV1 and PC3) and prostate cancer xenografts (LAPC-9 Androgen dependent and independent). Note expression of N-Cadherin in J82 and the androgen independent lines PC3, 22RV1 and LAPC-9 AI. N-Cadheirn is also expressed in LAPC-4 AI (not shown). Note that E Cadherin is downregulated in PC3 and J82, but not in the 22RV1 and 9AI cell lines/xenografts.

**Figure 8****: Real time PCR analysis of N-Cadherin expression.** N-Cadherin expression was evaluated in hormone refractory prostate cancer metastases and prostate cancer cell lines PC3, LNCaP, LAPC-4 AD and AI, LAPC 9 AD and AI, and 22RV 1. Levels of expression are normalized to PC3, a very high N-Cadherin expressing cell line. 22 RV1 expresses lower levels of N-Cadherin, which are detectable by Western. Note that five tumors express 4-25 fold (00-090EE and 01-046C) higher levels of N-Cadherin than PC3. Levels similar to PC3 are found in 16/21 cases.

**Figure 9****. LNCaP cells transduced with N-cadherin.** LNCaP cells transduced with N-cadherin express high levels ofN-Cadherin, leading to downregulation of E-Cadherin and striking morphological changes consistent with EMT. LNCaP-N-cadherin cells are highly invasive in vitro. There is no change in Akt in these PTEN null cells.

**Figure 10****. LNCaP-N-Cadherin cells in mice.** LNCaP-N-Cadherin cells in mice form tumors in castrate mice rapidly while control cells do not grow, consistent with conversion to an androgen independent phenotype.

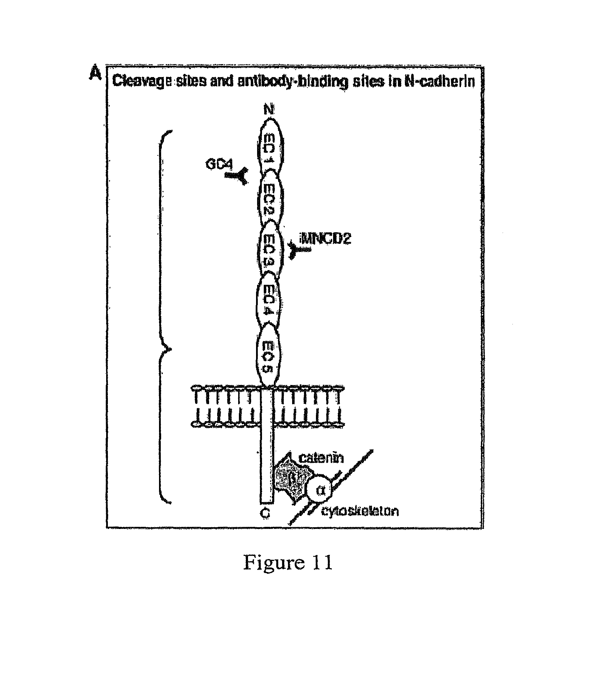

**Figure 11****. Cleavage sites and antibody binding sites in N-cadherin.**

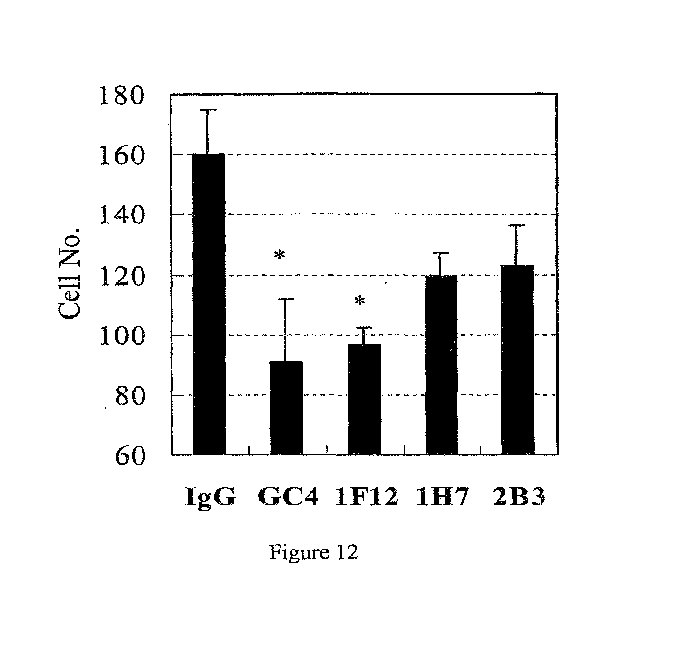

**Figure 12****. Screening of antibodies by effect on invasive activity in LNCaP-N-cadherin cells.**

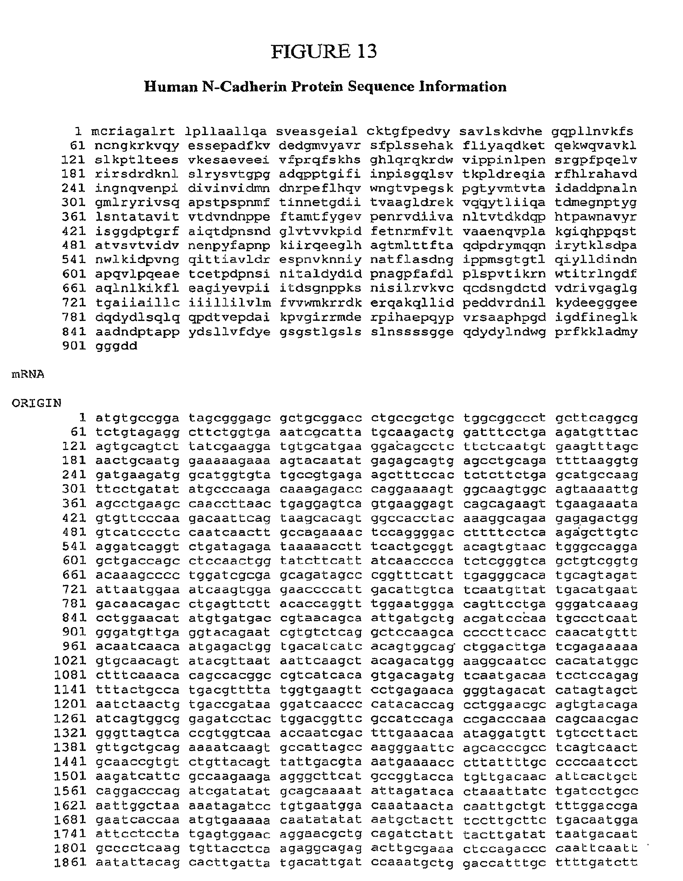

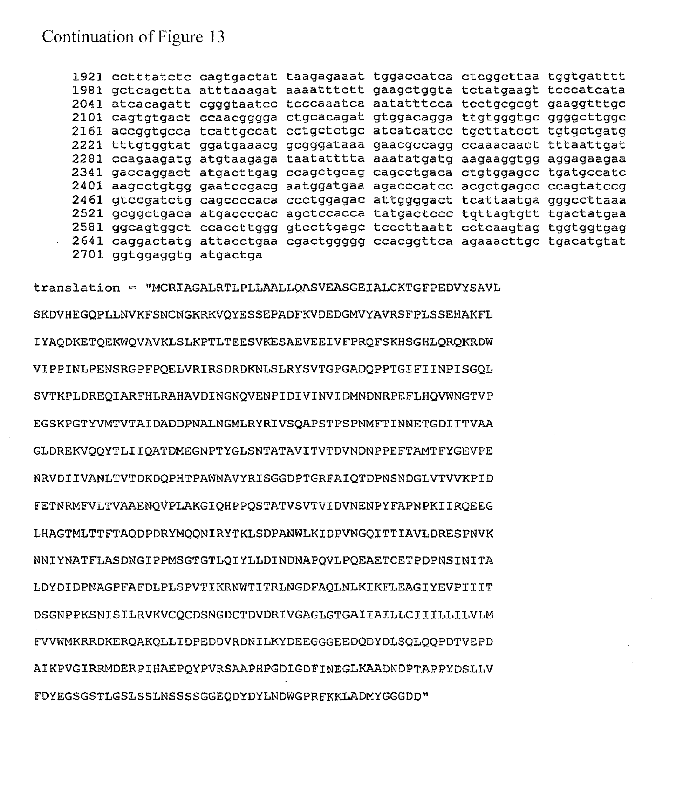

**Figure 13****. N-Cadherin nucleotide and amino acid sequence information.**

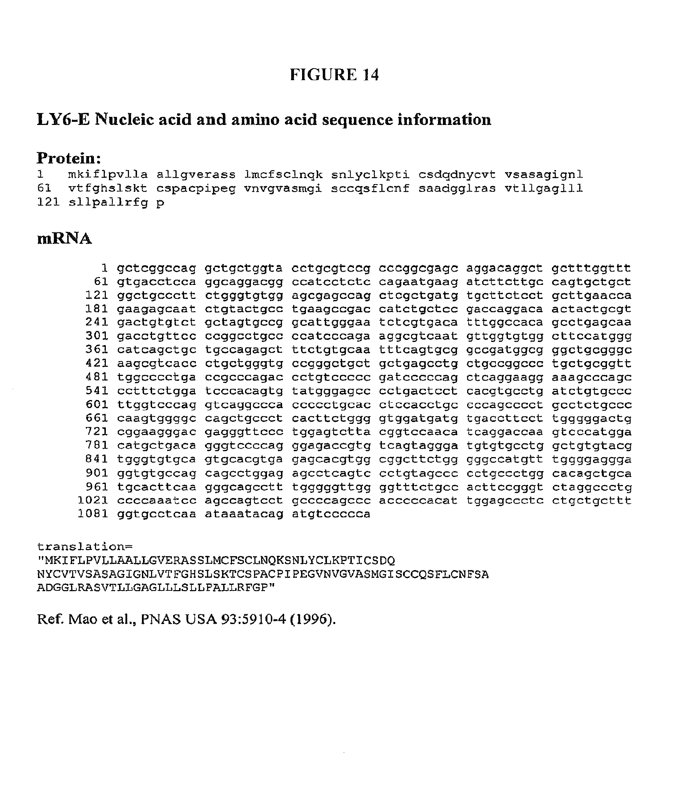

**Figure 14****. Ly6-E nucleotide and amino acid sequence information.**

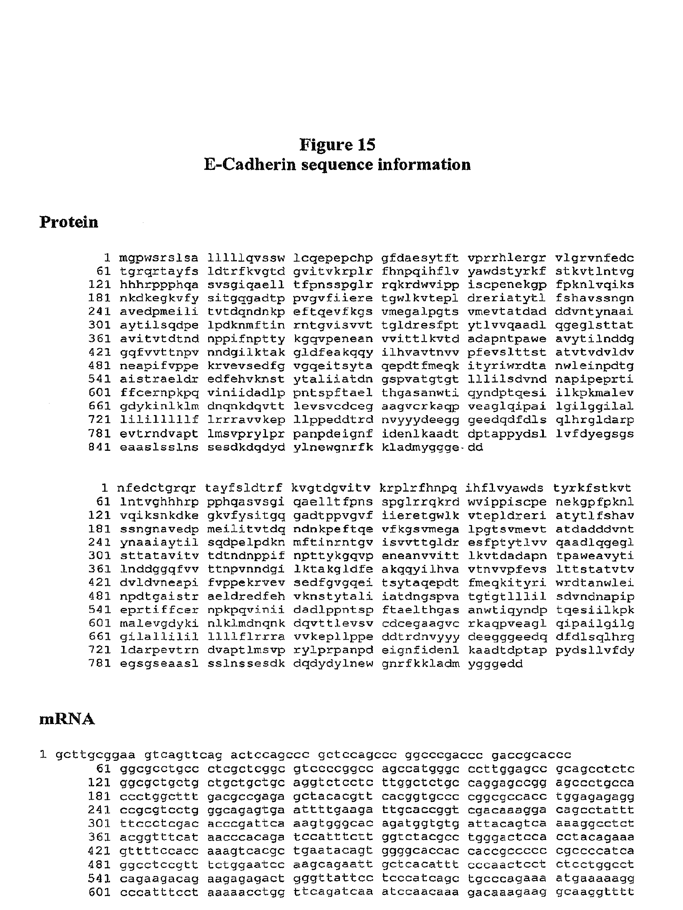

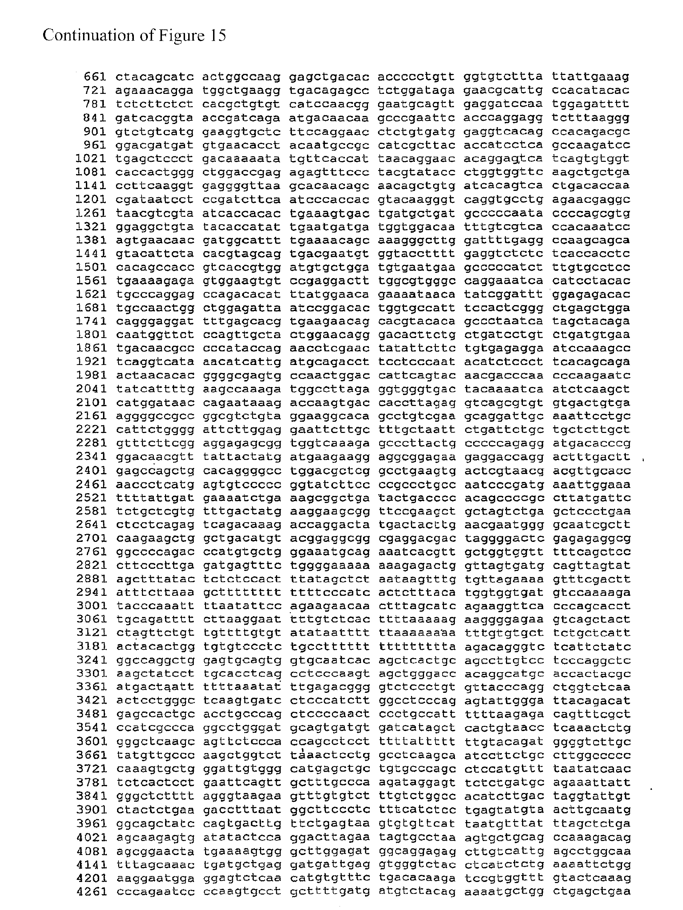

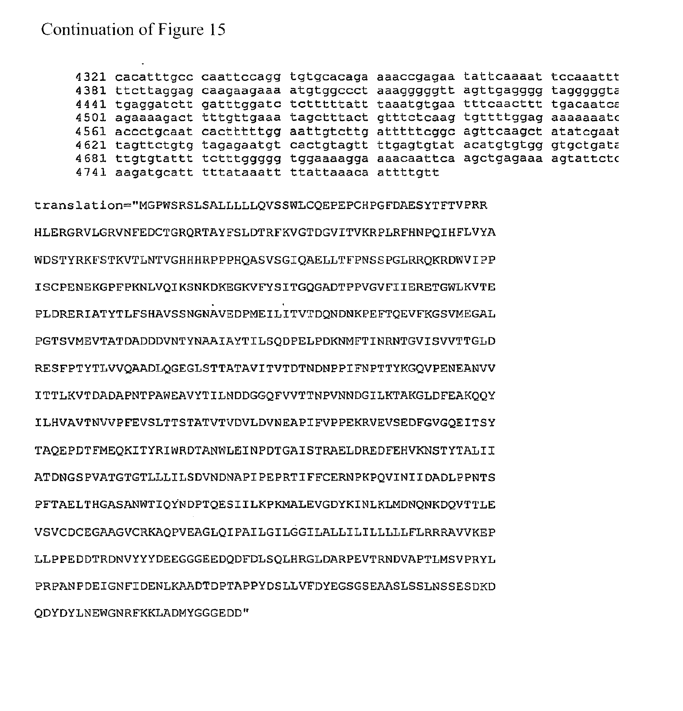

**Figure 15****. E-Cadherin nucleotide and amino acid sequence information.**

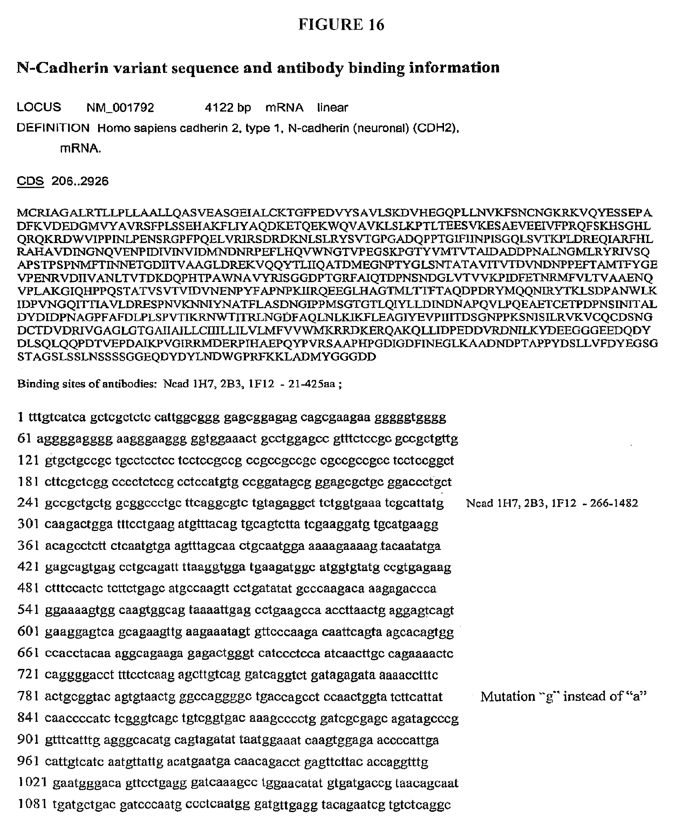

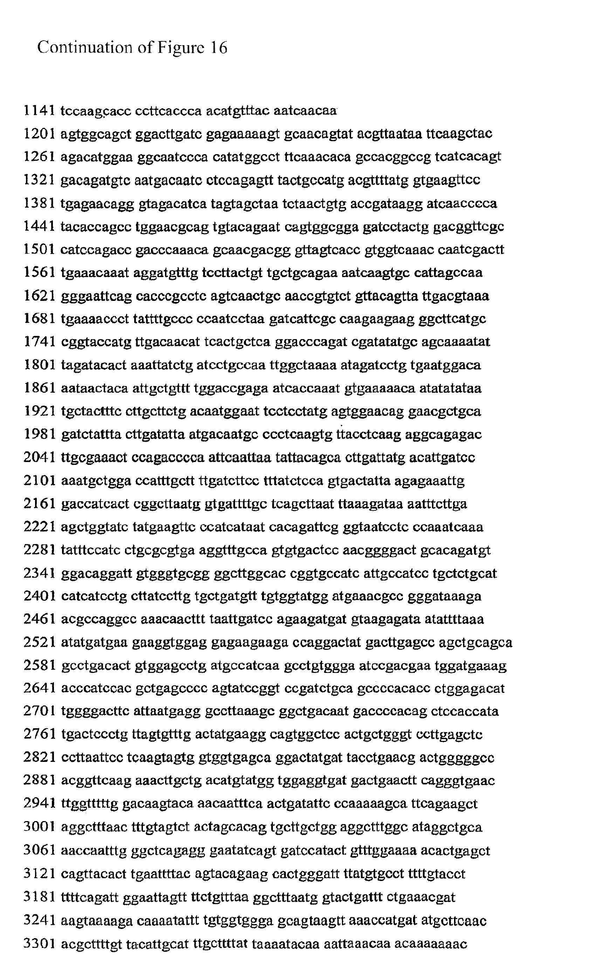

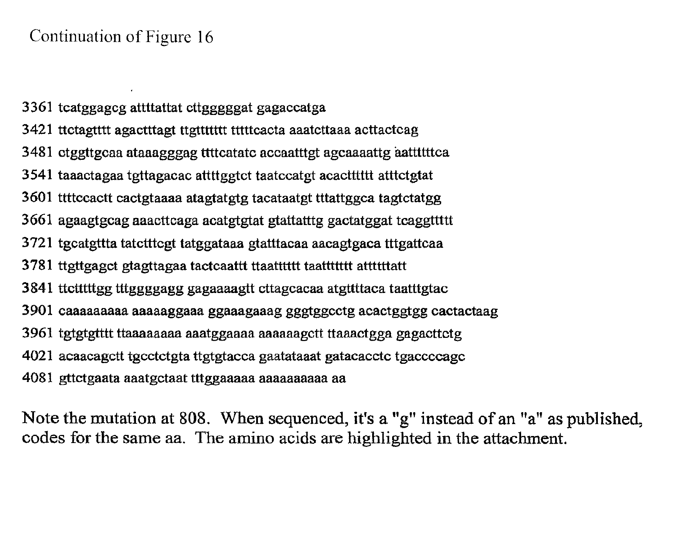

**Figure 16****. N-Cadherin variant sequence and antibody binding information**

### DETAILED DESCRIPTION OF THE INVENTION

We report here on the identification, characterization and validation of two gene products which are overexpressed in hormone refractory prostate cancer and bladder cancer. These gene products are N-Cadherin and Ly6-E.

This invention also relate to the discovery that invasive bladder and prostate cancers undergo an Epithelial to Mesenchymal Transition (EMT) characterized by upregulation of N-Cadherin and downregulation of E-Cadherin. We have also found a neutralizing N-Cadherin antibody can block bladder cancer cell invasion by reducing phosphorylated Akt expression and upregulating E-Cadherin, indicating that antibodies against such markers have utility in the treatment and prevention of cancer and, particularly, cancer metastases. In particular, we have found that N-Cadherin expression in bladder cancer is associated with activated Akt expression and invasive activity in Boyden chamber and in vitro reconstitution model, 2) N-Cadherin neutralization reduces invasion specifically by inhibiting Akt phosphorylation, restoring E-Cadherin to reverse EMT. PI3 Kinase inhibition does not restore E-Cadherin, suggesting N-Cadherin may signal through multiple pathways and that the N-Cadherin/E-Cadherin provides strong prognostic information.

With respect to prostate cancer, the invention relates to the findings that N-Cadherin promotes invasive and metastatic progression of prostate cancer and also promotes androgen independent growth:
● N-Cadherin is upregulated in androgen-independent xenografts
● -Cadherin expression common in hormone refractory tumors
● N-Cadherin expression promotes invasive and androgen-independent growth in androgen dependent prostate cancer cells
● Invasiveness associated with metalloproteinase expression
● Invasiveness reduced by antibody exposure

Accordingly, N-Cadherin is an especially promising therapeutic target. It is found on cell surfaces, overexpressed in many epithelial tumors, and is associated with invasion, metastasis and possibly androgen independence. Antibodies against N-cadherin therefore are a particularly preferred agent for use in treating epithelial cancers, including but not limited to urogenital cancers (bladder, prostate), and, more particularly, their invasive or metastasized forms. In some embodiments, monoclonal antibodies directed against an extracellular domain of N-cadherin are preferred. In further embodiments, the first extracellular domain (EC1), portions of the first and second domains, or fourth extracellular domain of N-cadherin are preferred in treating these cancers. In some embodiments, use of a antibody directed toward the extracellular domain 4 is particularly preferred in these treatments as this domain is found to be important in pro-motility and invasive potential (see, Kim et al, J Cell Biol. 151(6):1193-206 (2000), incorporated by reference in its entirety with respect to the definition of the various N-cadherin domains.

N-cadherin expression can contribute to prostate and bladder cancer invasion and metastasis as well as the progression of prostate caner to hormone refractory disease. N-Cadherin can be targeted therapeutically both alone and in combination with other small molecule inhibitors of mTOR and EGFR. Targeting N-Cadherin can help prevent or control invasive and metastatic prostate cancer.

### Definitions

"N-Cadherin, LY6-E, and E-Cadherin" refer to nucleic acids, e.g., gene, pre-mRNA, mRNA, and polypeptides, polymorphic variants, alleles, mutants, and interspecies homologs that: (1) have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of over a region of at least about 25, 50, 100, 200, 500, 1000, or more amino acids, to a polypeptide encoded by a respectively referenced nucleic acid or an amino acid sequence described herein, for example, as depicted in Figure 7, 8, and 9, respectively; (2) specifically bind to antibodies, e.g., polyclonal antibodies, raised against an immunogen comprising a referenced amino acid sequence as depicted in Figures 7, 8, and 9, respectively; immunogenic fragments respectively thereof, and conservatively modified variants respectively thereof; (3) specifically hybridize under stringent hybridization conditions to a nucleic acid encoding a referenced amino acid sequence as depicted in Figure s 7, 8, and 9, respectively, and conservatively modified variants respectively thereof; (4) have a nucleic acid sequence that has greater than about 95%, preferably greater than about 96%, 97%, 98%, 99%, or higher nucleotide sequence identity, preferably over a region of at least about 25, 50, 100, 150, 200, 250, 500, 1000, or more nucleotides, to a reference nucleic acid sequence as shown, respectively, in Figures 7, 8, and 9. A polynucleotide or polypeptide sequence is typically from a mammal including, but not limited to, primate, e.g., human; rodent, e.g., rat, mouse, hamster; cow, pig, horse, sheep, or any mammal. The nucleic acids and proteins of the invention include both naturally occurring or recombinant molecules.

"Cancer" refers to human cancers and carcinomas, sarcomas, adenocarcinomas, lymphomas, leukemias, etc., including solid tumors and lymphoid cancers, kidney, breast, lung, kidney, bladder, colon, ovarian, prostate, pancreas, stomach, brain, head and neck, skin, uterine, testicular, esophagus, and liver cancer, lymphoma, including non-Hodgkin's and Hodgkin's lymphoma, leukemia, and multiple myeloma. "Urogenital cancer" refers to human cancers of urinary tract and genital tissues, including but not limited to kidney, bladder, urinary tract, urethra, prostrate, penis, testicle, vulva, vagina, cervical and ovary tissues.

The cancer to be treated herein may be one characterized by excessive activation of N-cadherin or Ly6-E. In one embodiment of the invention, a diagnostic or prognostic assay will be performed to determine whether the patient's cancer is characterized by overexpression of N-cadherin or Ly6-E. Various assays for determining such amplification/overexpress ion are contemplated and include the immunohistochemistry, FISH and shed antigen assays, southern blotting, or PCR techniques. Moreover, the N-cadherin or Ly6-E over expression or amplification may be evaluated using an *in vivo* diagnostic assay, e.g. by administering a molecule (such as an antibody) which binds the molecule to be detected and is tagged with a detectable label (e.g. a radioactive isotope) and externally scanning the patient for localization of the label. In some embodiments, the cancer to be treated is not yet invasive, but overexpresses N-cadherin.

"Therapy resistant" cancers, tumor cells, and tumors refers to cancers that have become resistant or refractory to either or both apoptosis-mediated (*e*.*g*., through death receptor cell signaling, for example, Fas ligand receptor, TRAIL receptors, TNF-R1, chemotherapeutic drugs, radiation) and non-apoptosis mediated (e.g., toxic drugs, chemicals) cancer therapies, including chemotherapy, hormonal therapy, radiotherapy, and immunotherapy.

"Overexpression" refers to RNA or protein expression of N-Cadherin, LY6-E, and E-Cadherin in a test tissue sample that is significantly higher that RNA or protein expression ofN-Cadherin, LY6-E, and E-Cadherin, respectively, in a control tissue sample. In one embodiment, the tissue sample is autologous. Cancerous test tissue samples (e.g., bladder, prostate) associated with invasiveness, metastasis, hormone independent (e.g., androgen independence), or refractoriness to treatment or an increased likelihood of same typically have at least two fold higher expression ofN-Cadherin or LY6-E mRNA or protein, often up to three, four, five, eight, ten or more fold higher expression of N-Cadherin, or LY6-E in comparison to cancer tissues from patients who are less likely to progress to metastasis or to normal (i.e., non-cancer) tissue samples. Such differences may be readily apparent when viewing the bands of gels with approximately similarly loaded with test and controls samples. Prostate cancers expressing increased amounts ofN-Cadherin or Ly6-E are more likely to become invasive, metastasize, or progress to androgen independent or treatment refractory cancer. Various cutoffs are pertinent for N-Cadherin or Ly6-E positivity, since it is possible that a small percentage of N-Cadherin or Ly6-E positive cells in primary tumors may identify tumors with a high risk for recurrence and metastasis. The terms "overexpress," "overexpression" or "overexpressed" interchangeably refer to a gene that is transcribed or translated at a detectably greater level, usually in a cancer cell, in comparison to a normal cell. Overexpression therefore refers to both overexpression of protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., as in an increased enzyme hydrolysis of substrate. Overexpression can also be by 50%, 60%, 70%, 80%, 90% or more in comparison to a normal cell or comparison cell (e.g., a BPH cell).

The terms "cancer that overexpresses N-Cadherin or LY6-E" and "cancer associated with the overexpression of N-Cadherin or LY6-E" interchangeably refer to cancer cells or tissues that overexpress N-Cadherin or LY6-E in accordance with the above definition.

The terms "cancer-associated antigen" or "tumor-specific marker" or "tumor marker" interchangeably refers to a molecule (typically protein, carbohydrate or lipid) that is preferentially expressed in a cancer cell in comparison to a normal cell, and which is useful for the preferential targeting of a pharmacological agent to the cancer cell. A marker or antigen can be expressed on the cell surface or intracellularly. Oftentimes, a cancer-associated antigen is a molecule that is overexpressed or stabilized with minimal degradation in a cancer cell in comparison to a normal cell, for instance, 2-fold overexpression, 3-fold overexpression or more in comparison to a normal cell. Often times, a cancer-associated antigen is a molecule that is inappropriately synthesized in the cancer cell, for instance, a molecule that contains deletions, additions or mutations in comparison to the molecule expressed on a normal cell. Oftentimes, a cancer-associated antigen will be expressed exclusively in a cancer cell and not synthesized or expressed in a normal cell. Exemplified cell surface tumor markers include the proteins c-erbB-2 and human epidermal growth factor receptor (HER) for breast cancer, PSMA for prostate cancer, and carbohydrate mucins in numerous cancers, including breast, ovarian and colorectal. Exemplified intracellular tumor markers include; for example, mutated tumor suppressor or cell cycle proteins, including p53.

E-cadherin is conversely typically underexpressed in cancerous tissue samples in tissue samples from cancer patients which are likely to become invasive, metastasize, or progress to androgen independent or treatment refractory cancer. This underexpression may be two-fold, three-fold, four-fold, or at least five-fold. Such differences may be readily apparent when viewing the bands of gels with approximately similarly loaded with test and controls samples. The combined N-cadherin/E-cadherin values in cancers which are likely to become invasive, metastasize, or progress to androgen independent or treatment refractory cancer, are therefore even greater and can be at least two-fold, three-fold, four-fold, five-fold, ten-fold, or twenty-fold greater. Various cutoffs are pertinent for N-Cadherin positivity/e-cadherin negativity, since it is possible that a small percentage of N-Cadherin positive cells in primary tumors may identify tumors with a high risk for recurrence and metastasis.

An "agonist" refers to an agent that binds to a polypeptide or polynucleotide of the invention, stimulates, increases, activates, facilitates, enhances activation, sensitizes or up regulates the activity or expression of a polypeptide or polynucleotide of the invention.

An "antagonist" refers to an agent that inhibits expression of a polypeptide or polynucleotide of the invention or binds to, partially or totally blocks stimulation, decreases, prevents, delays activation, inactivates, desensitizes, or down regulates the activity of a polypeptide or polynucleotide of the invention.

"Inhibitors," "activators," and "modulators" of expression or of activity are used to refer to inhibitory, activating, or modulating molecules, respectively, identified using in vitro and in vivo assays for expression or activity, e.g., ligands, agonists, antagonists, and their homologs and mimetics. The term "modulator" includes inhibitors and activators. Inhibitors are agents that, e.g., inhibit expression of a polypeptide or polynucleotide of the invention or bind to, partially or totally block stimulation or enzymatic activity, decrease, prevent, delay activation, inactivate, desensitize, or down regulate the activity of a polypeptide or polynucleotide of the invention, e.g., antagonists. Activators are agents that, e.g., induce or activate the expression of a polypeptide or polynucleotide of the invention or bind to, stimulate, increase, open, activate, facilitate, enhance activation or enzymatic activity, sensitize or up regulate the activity of a polypeptide or polynucleotide of the invention, e.g., agonists. Modulators include naturally occurring and synthetic ligands, antagonists, agonists, small chemical molecules and the like. Assays to identify inhibitors and activators include, e.g., applying putative modulator compounds to cells, in the presence or absence of a polypeptide or polynucleotide of the invention and then determining the functional effects on a polypeptide or polynucleotide of the invention activity. Samples or assays comprising a polypeptide or polynucleotide of the invention that are treated with a potential activator, inhibitor, or modulator are compared to control samples without the inhibitor, activator, or modulator to examine the extent of effect. Control samples (untreated with modulators) are assigned a relative activity value of 100%. Inhibition is achieved when the activity value of a polypeptide or polynucleotide of the invention relative to the control is about 80%, optionally 50% or 25-1%. Activation is achieved when the activity value of a polypeptide or polynucleotide of the invention relative to the control is 110%, optionally 150%, optionally 200-500%, or 1000-3000% higher.

The term "test compound" or "drug candidate" or "modulator" or grammatical equivalents as used herein describes any molecule, either naturally occurring or synthetic, e.g., protein, oligopeptide (e.g., from about 5 to about 25 amino acids in length, preferably from about 10 to 20 or 12 to 18 amino acids in length, preferably 12, 15, or 18 amino acids in length), small organic molecule, polysaccharide, lipid, fatty acid, polynucleotide, RNAi, siRNA, antibody, oligonucleotide, etc. The test compound can be in the form of a library of test compounds, such as a combinatorial or randomized library that provides a sufficient range of diversity. Test compounds are optionally linked to a fusion partner, e.g., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, e.g., inhibiting activity, creating variants of the lead compound, and evaluating the property and activity of those variant compounds. Often, high throughput screening (HTS) methods are employed for such an analysis.

A "small organic molecule" refers to an organic molecule, either naturally occurring or synthetic, that has a molecular weight of more than about 50 Daltons and less than about 2500 Daltons, preferably less than about 2000 Daltons, preferably between about 100 to about 1000 Daltons, more preferably between about 200 to about 500 Daltons.

Cytotoxic agents include "cell-cycle-specific" or "antimitotic" or "cytoskeletal-interacting" drugs. These terms interchangeably refer to any pharmacological agent that blocks cells in mitosis. Such agents are useful in chemotherapy. Generally, cell-cycle-specific-drugs bind to the cytoskeletal protein tubulin and block the ability of tubulin to polymerize into microtubules, resulting in the arrest of cell division at metaphase. Exemplified cell-cycle-specific drugs include vinca alkaloids, taxanes, colchicine, and podophyllotoxin. Exemplified vinca alkaloids include vinblastine, vincristine, vindesine and vinorelbine. Exemplifed taxanes include paclitaxel and docetaxel. Another example of a cytoskeletal-interacting drug includes 2-methoxyestradiol.

An "siRNA" or "RNAi" refers to a nucleic acid that forms a double stranded RNA, which double stranded RNA has the ability to reduce or inhibit expression of a gene or target gene when the siRNA expressed in the same cell as the gene or target gene. "siRNA" or "RNAi" thus refers to the double stranded RNA formed by the complementary strands. The complementary portions of the siRNA that hybridize to form the double stranded molecule typically have substantial or complete identity. In one embodiment, an siRNA refers to a nucleic acid that has substantial or complete identity to a target gene and forms a double stranded siRNA. Typically, the siRNA is at least about 15-50 nucleotides in length (e.g., each complementary sequence of the double stranded siRNA is 15-50 nucleotides in length, and the double stranded siRNA is about 15-50 base pairs in length, preferable about preferably about 20-30 base nucleotides, preferably about 20-25 or about 24-29 nucleotides in length, e.g., 20, 21, 22, 23, 24, 25, 26, 27, 28, 29, or 30 nucleotides in length.

The design and making of siRNA molecules and vectors are well known to those of ordinary skill in the art. For instance, an efficient process for designing a suitable siRNA is to start at the AUG start codon of the mRNA transcript (e.g., see, Figure 5) and scan for AA dinucleotide sequences (see, Elbashir et al. EMBO J 20: 6877-6888 (2001). Each AA and the 3' adjacent nucleotides are potential siRNA target sites. The length of the adjacent site sequence will determine the length of the siRNA. For instance, 19 adjacent sites would give a 21 Nucleotide long siRNA siRNAs with 3' overhanging UU dinucleotides are often the most effective. This approach is also compatible with using RNA pol III to transcribe hairpin siRNAs. RNA pol III terminates transcription at 4-6 nucleotide poly(T) tracts to create RNA molecules having a short poly(U) tail. However, siRNAs with other 3` terminal dinucleotide overhangs can also effectively induce RNAi and the sequence may be empirically selected. For selectivity, target sequences with more than 16-17 contiguous base pairs of homology to other coding sequences can be avoided by conducting a BLAST search *(see,* www.ncbi.nlm.nih.gov/BLAST).

The siRNA can be administered directly or an siRNA expression vectors can be used to induce RNAi can have different design criteria. A vector can have inserted two inverted repeats separated by a short spacer sequence and ending with a string of T's which serve to terminate transcription. The expressed RNA transcript is predicted to fold into a short hairpin siRNA. The selection of siRNA target sequence, the length of the inverted repeats that encode the stem of a putative hairpin, the order of the inverted repeats, the length and composition of the spacer sequence that encodes the loop of the hairpin, and the presence or absence of 5'-overhangs, can vary. A preferred order of the siRNA expression cassette is sense strand, short spacer, and antisense strand. Hairpin siRNAs with these various stem lengths (e.g., 15 to 30) can be suitable. The length of the loops linking sense and antisense strands of the hairpin siRNA can have varying lengths (e.g., 3 to 9 nucleotides, or longer). The vectors may contain promoters and expression enhancers or other regulatory elements which are operably linked to the nucleotide sequence encoding the siRNA. The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers. These control elements may be designed to allow the clinician to turn off or on the expression of the gene by adding or controlling external factors to which the regulatory elements are responsive.

Construction of suitable vectors containing the desired therapeutic gene coding and control sequences employs standard ligation and restriction techniques, which are well understood in the art (see Maniatis et al., in Molecular Cloning: A Laboratory Manual, Cold Spring Harbor Laboratory, New York (1982)). Isolated plasmids, DNA sequences, or synthesized oligonucleotides are cleaved, tailored, and re-ligated in the form desired.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are near each other, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Determining the functional effect" refers to assaying for a compound that increases or decreases a parameter that is indirectly or directly under the influence of a polynucleotide or polypeptide of the invention, e.g., measuring physical and chemical or phenotypic effects. Such functional effects can be measured by any means known to those skilled in the art, e.g., changes in spectroscopic (e.g., fluorescence, absorbance, refractive index), hydrodynamic (e.g., shape), chromatographic, or solubility properties for the protein; measuring inducible markers or transcriptional activation of the protein; measuring binding activity or binding assays, e.g. binding to antibodies; measuring changes in ligand binding affinity; measurement of calcium influx; measurement of the accumulation of an enzymatic product of a polypeptide of the invention or depletion of an substrate; changes in enzymatic activity, e,g., kinase activity, measurement of changes in protein levels of a polypeptide of the invention; measurement of RNA stability; G-protein binding; GPCR phosphorylation or dephosphorylation; signal transduction, e.g., receptor-ligand interactions, second messenger concentrations (e.g., cAMP, IP3, or intracellular Ca2+); identification of down stream or reporter gene expression (CAT, luciferase, β-gal, GFP and the like), e.g., via chemiluminescence, fluorescence, colorimetric reactions, antibody binding, inducible markers, and ligand binding assays..

Samples or assays comprising a nucleic acid or protein disclosed herein that are treated with a potential activator, inhibitor, or modulator are compared to control samples without the inhibitor, activator, or modulator to examine the extent of inhibition. Control samples (untreated with inhibitors) are assigned a relative protein activity value of 100%. Inhibition is achieved when the activity value relative to the control is about 80%, preferably 50%, more preferably 25-0%. Activation is achieved when the activity value relative to the control (untreated with activators) is 110%, more preferably 150%, more preferably 200-500% (i.e., two to five fold higher relative to the control), more preferably 1000-3000% higher.

"Biological sample" includes sections of tissues such as biopsy and autopsy samples, and frozen sections taken for histological purposes. Such samples include blood and blood fractions or products (e.g., serum, plasma, platelets, red blood cells, and the like), sputum, tissue, cultured cells, e.g., primary cultures, explants, and transformed cells, stool, urine, etc. A biological sample is typically obtained from a eukaryotic organism, most preferably a mammal such as a primate e.g., chimpanzee or human; cow; dog; cat; a rodent, e.g., guinea pig, rat, Mouse; rabbit; or a bird; reptile; or fish.

A "biopsy" refers to the process of removing a tissue sample for diagnostic or prognostic evaluation, and to the tissue specimen itself. Any biopsy technique known in the art can be applied to the diagnostic and prognostic methods of the present invention. The biopsy technique applied will depend on the tissue type to be evaluated (*i*.*e*., prostate, lymph node, liver, bone marrow, blood cell), the size and type of the tumor (*i*.*e*., solid or suspended (i.e., blood or ascites)), among other factors. Representative biopsy techniques include excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue that includes a cross-sectional diameter of the tumor. A diagnosis or prognosis made by endoscopy or fluoroscopy can require a "core-needle biopsy" of the tumor mass, or a "fine-needle aspiration biopsy" which generally obtains a suspension of cells from within the tumor mass. Biopsy techniques are discussed, for example, in Harrison's Principles of Internal Medicine, Kasper, et al., eds., 16th ed., 2005, Chapter 70, and throughout Part V.

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection *(see, e.g.,* NCBI web site http://www.ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection *(see, e.g.,* Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.*, *supra*)*.* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of, an expectation (E) of 10, M=5, N=-4 and a comparison of both strands. For amino acid sequences, the BLASTP program uses as defaults a wordlength of 3, and expectation (E) of 10, and the BLOSUM62 scoring matrix *(see* Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide.

A particular nucleic acid sequence also implicitly encompasses "splice variants." Similarly, a particular protein encoded by a nucleic acid implicitly encompasses any protein encoded by a splice variant of that nucleic acid. "Splice variants," as the name suggests, are products of alternative splicing of a gene. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, including recombinant forms of the splice products, are included in this definition. An example of potassium channel splice variants is discussed in Leicher, et al., J, Biol. Chem. 273(52):35095-35101 (1998).

The terms "polypeptides," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) *(see, e.g.,* Creighton, Proteins (1984)).

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (e.g., as commonly used in an ELISA), biotin, digoxigenin, or haptens and proteins which can be made detectable, e.g., by incorporating a radiolabel into the peptide or used to detect antibodies specifically reactive with the peptide.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C.

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, e.g., and Current Protocols in Molecular Biology, ed. Ausubel, et al., John Wiley & Sons.

For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec - 2 min., an annealing phase lasting 30 sec. - 2 min., and an extension phase of about 72°C for 1 - 2 min. Protocols and guidelines for low and high stringency amplification reactions are provided, e.g., in Innis et al. (1990) PCR Protocols, A Guide to Methods and Applications, Academic Press, Inc. N.Y.).

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region *(see* Fundamental Immunology (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries *(see, e.g.,* McCafferty et al., Nature 348:552-554 (1990))

For preparation of suitable antibodies of the invention and for use according to the invention, e.g., recombinant, monoclonal, or polyclonal antibodies, many techniques known in the art can be used *(see, e.g.,* Kohler & Milstein, Nature 256:495-497 (1975); Kozbor et al., Immunology Today 4: 72 (1983); Cole et al., pp. 77-96 in Monoclonal Antibodies and Cancer Therapy, Alan R. Liss, Inc. (1985); Coligan, Current Protocols in Immunology (1991); Harlow & Lane, Antibodies, A Laboratory Manual (1988); and Goding, Monoclonal Antibodies: Principles and Practice (2d ed. 1986)). The genes encoding the heavy and light chains of an antibody of interest can be cloned from a cell, e.g., the genes encoding a monoclonal antibody can be cloned from a hybridoma and used to produce a recombinant monoclonal antibody. Gene libraries encoding heavy and light chains of monoclonal antibodies can also be made from hybridoma or plasma cells. Random combinations of the heavy and light chain gene products generate a large pool of antibodies with different antigenic specificity *(see, e.g.,* Kuby, Immunology (3rd ed. 1997)). Techniques for the production of single chain antibodies or recombinant antibodies (U.S. Patent 4,946,778, U.S. Patent No. 4,816,567) can be adapted to produce antibodies to polypeptides of this invention. Also, transgenic mice, or other organisms such as other mammals, may be used to express humanized or human antibodies *(see, e.g.,* U.S. Patent Nos. 5,545,807; 5,545,806; 5,569,825; 5,625,126; 5,633,425; 5,661,016, Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); and Lonberg & Huszar, Intern. Rev. Immunol. 13:65-93 (1995)). Alternatively, phage display technology can be used to identify antibodies and heteromeric Fab fragments that specifically bind to selected antigens *(see, e.g.,* McCafferty et al., Nature 348:552-554 (1990); Marks et al., Biotechnology 10:779-783 (1992)). Antibodies can also be made bispecific, i.e., able to recognize two different antigens *(see, e.g.,* WO 93/08829, Traunecker et al., EMBO J. 10:3655-3659 (1991); and Suresh et al., Methods in Enzymology 121:210 (1986)). Antibodies can also be heteroconjugates, e.g., two covalently joined antibodies, or immunotoxins *(see, e.g.,* U.S. Patent No. 4,676,980, WO 91/00360; WO 92/200373; and EP 03089).

Methods for humanizing or primatizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers *(see, e.g.,* Jones et al., Nature 321:522-525 (1986); Riechmann et al., Nature 332:323-327 (1988); Verhoeyen et al., Science 239:1534-1536 (1988) and Presta, Curr. Op. Struct. Biol. 2:593-596 (1992)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. The preferred antibodies of, and for use according to the invention include humanized and/or chimeric monoclonal antibodies.

In one embodiment, the antibody is conjugated to an "effector" moiety. The effector moiety can be any number of molecules, including labeling moieties such as radioactive labels or fluorescent labels, or can be a therapeutic moiety. In one aspect the antibody modulates the activity of the protein. Such effector moieties include, but are not limited to, an anti-tumor drug, a toxin, a radioactive agent, a cytokine, a second antibody or an enzyme. Further, the invention provides an embodiment wherein the antibody of the invention is linked to an enzyme that converts a prodrug into a cytotoxic agent.

The immunoconjugate can be used for targeting the effector moiety to a N-cadherin or Ly6-E positive cell, particularly cells, which overexpress the N-cadherin or Ly6 protein. Such differences can be readily apparent when viewing the bands of gels with approximately similarly loaded with test and controls samples.. Examples of cytotoxic agents include, but are not limited to ricin, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, and glucocorticoid and other chemotherapeutic agents, as well as radioisotopes. Suitable detectable markers include, but are not limited to, a radioisotope, a fluorescent compound, a bioluminescent compound, chemiluminescent compound, a metal chelator or an enzyme.

In some embodiments, the invention provides antibodies to N-cadherin or Ly6-E. N-cadherin or Ly6-E antibodies may be used systemically to treat cancer (e.g., prostate or bladder cancer) alone or when conjugated with an effector moiety. N-cadherin or Ly6-E antibodies conjugated with toxic agents, such as ricin, as well as unconjugated antibodies may be useful therapeutic agents naturally targeted to N-cadherin or Ly6-E -bearing prostate cancer cells. Such antibodies can be useful in blocking invasiveness. Suitable N-cadherin antibodies for use according to the invention include, but are not limited to, GC4 1H7, 1F12, 2B3.

Additionally, the recombinant protein of the invention comprising the antigen-binding region of any of the monoclonal antibodies of the invention can be used to treat cancer. In such a situation, the antigen-binding region of the recombinant protein is joined to at least a functionally active portion of a second protein having therapeutic activity. The second protein can include, but is not limited to, an enzyme, lymphokine, oncostatin or toxin. Suitable toxins include doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, ricin, abrin, glucocorticoid and radioisotopes.

Techniques for conjugating therapeutic agents to antibodies are well known (see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery"in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review" in Monoclonal Antibodies '84: Biological And Clinical Applications, Pinchera et al. (eds.), pp. 475-506 (1985); and Thorpe et al., "The Preparation And Cytotoxic Properties Of Antibody-Toxin Conjugates", Immunol. Rev., 62:119-58 (1982)).

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics. Thus, under designated immunoassay conditions, the specified antibodies bind to a particular protein at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein *(see, e.g.,* Harlow & Lane, Using Antibodies, A Laboratory Manual (1998) for a description of immunoassay formats and conditions that can be used to determine specific immunoreactivity).

By "therapeutically effective dose or amount" herein is meant a dose that produces effects for which it is administered. The exact dose and formulation will depend on the purpose of the treatment, and will be ascertainable by one skilled in the art using known techniques *(see, e.g.,* Lieberman, Pharmaceutical Dosage Forms (vols. 1-3, 1992); Lloyd, The Art, Science and Technology of Pharmaceutical Compounding (1999); Remington: The Science and Practice of Pharmacy, 20th Edition, Gennaro, Editor (2003), and Pickar, Dosage Calculations (1999)).

The term "pharmaceutically acceptable salts" or "pharmaceutically acceptable carrier" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like *(see, e.g.,* Berge et al., Journal of Pharmaceutical Science 66:1-19 (1977)). Certain specific compounds of the present invention contain both basic and acidic functionalities that allow the compounds to be converted into either base or acid addition salts. Other pharmaceutically acceptable carriers known to those of skill in the art are suitable for the present invention.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an ex *vivo* environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are all intended to be encompassed within the scope of the present invention.

Epithelial to Mesenchymal Transition (EMT) refers to the acquisition of stromal features by epithelial tumor cells. In cancer, EMT is associated with invasive and motile behavior and may be central process underlying metastasis. EMT is associated with poor prognosis and is mediated by multiple transcription factors, such as, SNAIL, SLUG and TWIST.
E-Cadherin is a cell surface protein involved in epithelial cell-cell adhesion which is commonly lost in invasive and metastatic solid tumors.

### Detailed Embodiments

The present invention provides methods of diagnosis and providing a prognosis for individuals at risk for a cancer that overexpresses a N-Cadherin or LY6-E protein or mRNA transcript, particularly urogenital cancers including prostate and/or bladder cancer. The methods generally comprise contacting a test tissue sample from an individual at risk of having a cancer that overexpresses a N-Cadherin or LY6-E protein or mRNA transcript with an antibody that specifically binds to a N-Cadherin or LY6-E protein; and determining the presence or absence of a N-Cadherin or LY6-E protein in the test tissue sample in comparison to a control tissue sample from an individual known to be negative for a cancer that overexpresses a N-Cadherin or LY6-E protein or mRNA transcript. Typically, the tissue sample is serum, but can also be a tissue from a biopsy, particularly from a urogenital tissue including prostate tissue or bladder tissue. Usually, the antibody is a monoclonal antibody. A positive diagnosis for a cancer that overexpresses a N-Cadherin or LY6-E protein or mRNA transcript is indicated when a higher level of N-Cadherin or LY6-E protein is detected in a test tissue sample in comparison to a control tissue sample from an individual known not to have cancer, for example, 10%, 20%, 30%, 40%, 50%, 60%, 70%, 80%, 90%, 100%, 2-fold, 3-fold, 4-fold higher or more. The detection methods can be carried out, for example, using standard ELISA techniques known in the art *(reviewed in* Gosling, Immunoassays: A Practical Approach, 2000, Oxford University Press). Detection is accomplished by labeling a primary antibody or a secondary antibody with, for example, a radioactive isotope, a fluorescent label, an enzyme or any other detectable label known in the art.

In another embodiment, invention provides methods of diagnosis and providing a prognosis for individuals at risk for a cancer that overexpresses a N-Cadherin or LY6-E protein or mRNA transcript, particularly a prostate or bladder cancer, by contacting a test tissue sample from an individual at risk of having a cancer that overexpresses a N-Cadherin or LY6-E protein or mRNA transcript with a primer set of a first oligonucleotide and a second oligonucleotide that each specifically hybridize to a N-Cadherin or LY6-E nucleic acid; amplifying the N-Cadherin or LY6-E nucleic acid in the sample; and determining the presence or absence of the N-Cadherin or LY6-E nucleic acid in the test tissue sample in comparison to a control tissue sample from an individual known to be negative for a cancer that overexpresses a N-Cadherin or LY6-E protein or mRNA transcript. Again, usually the tissue sample is serum, but can also be a tissue from a biopsy, particularly a urogenital tissue including a prostate or bladder tissue. A positive diagnosis for a cancer that overexpresses a N-Cadherin or LY6-E protein or mRNA transcript is indicated when a higher level of N-Cadherin or LY6-E transcribed RNA is detected in a test tissue sample in comparison to a control tissue sample from an individual known not to have cancer.

The invention also provides methods for improving the response to cancer therapy in a cancer that overexpresses a N-Cadherin or LY6-E protein or mRNA transcript by administering a therapeutically effective amount of a compound that inhibits the binding of N-Cadherin or LY6-E protein to, respectively, a N-Cadherin or LY6-E receptor on a cell of the cancer tumor tissue. In some embodiments the methods of inhibiting N-Cadherin or LY6-E binding to its receptor are carried out concurrently with another anticancer therapy, including, for example, known chemotherapeutics, immunotherapeutics, and radiotherapy for the reversal of resistance, tumor progression, and metastasis.

The present invention further provides methods of inhibiting the growth of and promoting the regression of a tumor that overexpresses N-Cadherin or LY6-E protein, the methods comprising inhibiting the binding of N-Cadherin or LY6-E protein to, respectively, a N-Cadherin or LY6-E receptor on a cell of the tumor tissue. The methods can be carried out by administering to an individual in need thereof a sufficient amount of a compound that inhibits the binding of a N-Cadherin or LY6-E protein to respectively, a N-Cadherin or LY6-E receptor. In some embodiments, the compound specifically binds to a N-Cadherin or LY6-E protein. In some embodiments, the compound specifically binds to a N-Cadherin or LY6-E receptor. In some embodiments, the compound prevents the transcription or the translation of a N-Cadherin or LY6-E protein. The methods find particular use in treating prostate and bladder cancer. In some embodiments, the compound comprises a polypeptide, including an antibody or an analog or fragment of a N-Cadherin or LY6-E polypeptide.

The methods find particular application in the diagnosis, prognosis and treatment of prostate and bladder cancers. In certain embodiments the methods are applied to hormone refractory or therapy resistant cancers. In certain embodiments the methods are applied to metastatic cancers. For example comparisons of differential expression of a N-Cadherin or LY6-E protein and/or mRNA can be used to determine the stage of cancer of an individual having a cancer that overexpresses a N-Cadherin or LY6-E protein or mRNA transcript.

Treatment will generally involve the repeated administration of the anti- N-Cadherin or -LY6-E antibodies, immunoconjugates, inhibitors, and siRNA preparations via an acceptable route of administration such as intravenous injection (IV), at an effective dose. Dosages will depend upon various factors generally appreciated by those of skill in the art, including without limitation the type of cancer and the severity, grade, or stage of the cancer, the binding affinity and half life of the agents used, the degree of N-Cadherin or LY6-E expression in the patient, the extent of circulating shed N-Cadherin or LY6-E antigen, the desired steady-state antibody concentration level, frequency of treatment, and the influence of chemotherapeutic agents used in combination with the treatment method of the invention. Typical daily doses may range from about 0.1 to 100 mg/kg. Doses in the range of 10-500 mg of the mAb or immunoconjugates per week may be effective and well tolerated, although even higher weekly doses may be appropriate and/or well tolerated. The principal determining factor in defining the appropriate dose is the amount of a particular agent necessary to be therapeutically effective in a particular context. Repeated administrations may be required in order to achieve tumor inhibition or regression. Initial loading doses may be higher. The initial loading dose may be administered as an infusion. Periodic maintenance doses may be administered similarly, provided the initial dose is well tolerated.

Direct administration of the agents is also possible and may have advantages in certain contexts. For example, for the treatment of bladder carcinoma,the agents may be injected directly into the bladder. Because agents administered directly to bladder will be cleared from the patient rapidly, it may be possible to use non-human or chimeric antibodies effectively without significant complications of antigenicity.

The invention further provides vaccines formulated to contain a N-Cadherin or LY6-E protein or fragment thereof. The use of a tumor antigen in a vaccine for generating humoral and cell-mediated immunity for use in anti-cancer therapy is well known in the art and, for example, has been employed in prostate cancer using human PSMA and rodent PAP immunogens (Hodge et al., 1995, Int. J. Cancer 63: 231-237; Fong et al., 1997, J. Immunol. 159: 3113-3117). Such methods can be readily practiced by employing a N-Cadherin or LY6-E protein, or fragment thereof, or a N-Cadherin or LY6-E -encoding nucleic acid molecule and recombinant vectors capable of expressing and appropriately presenting the N-Cadherin or LY6-E immunogen.

For example, viral gene delivery systems may be used to deliver a N-Cadherin or LY6-E -encoding nucleic acid molecule. Various viral gene delivery systems which can be used in the practice of this aspect of the invention include, but are not limited to, vaccinia, fowlpox, canarypox, adenovirus, influenza, poliovirus, adeno-associated virus, lentivirus, and sindbus virus (Restifo, 1996, Curr. Opin. Immunol. 8: 658-663). Non-viral delivery systems may also be employed by using naked DNA encoding a N-Cadherin or LY6-E protein or fragment thereof introduced into the patient (e.g., intramuscularly) to induce an anti-tumor response. In one embodiment, the full-length human N-Cadherin or LY6-E cDNA may be employed. In another embodiment, N-Cadherin or LY6-E nucleic acid molecules encoding specific cytotoxic T lymphocyte (CTL) epitopes may be employed. CTL epitopes can be determined using specific algorithms (e.g., Epimer, Brown University) to identify peptides within a N-cadherin or Ly6-E protein which are capable of optimally binding to specified HLA alleles.

Various ex vivo strategies may also be employed. One approach involves the use of dendritic cells to present N-Cadherin or LY6-E antigen to a patient's immune system. Dendritic cells express MHC class I and II, B7 costimulator, and IL-12, and are thus highly specialized antigen presenting cells. In prostate cancer, autologous dendritic cells pulsed with peptides of the N-Cadherin or LY6-E can be used to stimulate prostate cancer patients' immune systems (Tjoa et al., 1996, Prostate 28: 65-69; Murphy et al., 1996, Prostate 29: 371-380). Dendritic cells can be used to present N-Cadherin or LY6-E peptides to T cells in the context of MHC class I and II molecules. In one embodiment, autologous dendritic cells are pulsed with N-Cadherin or LY6-E peptides capable of binding to MHC molecules. In another embodiment, dendritic cells are pulsed with the complete N-Cadherin or LY6-E protein. Yet another embodiment involves engineering the overexpression of the N-Cadherin or LY6-E gene in dendritic cells using various implementing vectors known in the art, such as adenovirus (Arthur et al., 1997, Cancer Gene Ther. 4: 17-25), retrovirus (Henderson et al., 1996, Cancer Res. 56: 3763-3770), lentivirus, adeno-associated virus, DNA transfection (Ribas et al., 1997, Cancer Res. 57: 2865-2869), and tumor-derived RNA transfection (Ashley et al., 1997, J. Exp. Med. 186: 1177-1182).

Anti-idiotypic anti- N-Cadherin or -LY6-E antibodies can also be used in anti-cancer therapy as a vaccine for inducing an immune response to cells expressing a N-Cadherin or LY6-E protein, respectively. Specifically, the generation of anri-idiotypic antibodies is well known in the art and can readily be adapted to generate anti-idiotypic anti-N-Cadherin or-LY6-E antibodies that respectively mimic an epitope on a N-Cadherin or LY6-E protein (see, for example, Wagner et al., 1997, Hybridoma 16: 33-40; Foon et al., 1995, J Clin Invest 96: 334-342; Herlyn et al., 1996, Cancer Immunol Immunother 43: 65-76). Such an anti-idiotypic antibody can be used in anti-idiotypic therapy as presently practiced with other anti-idiotypic antibodies directed against tumor antigens.

Genetic immunization methods may be employed to generate prophylactic or therapeutic humoral and cellular immune responses directed against cancer cells expressing N-Cadherin or LY6-E. Using the N-Cadherin or LY6-E -encoding DNA molecules described herein, constructs comprising DNA encoding a N-Cadherin or LY6-E protein/immunogen and appropriate regulatory sequences may be injected directly into muscle or skin of an individual, such that the cells of the muscle or skin take-up the construct and express the encoded N-Cadherin or LY6-E protein/immunogen. The N-Cadherin or LY6-E protein/immunogen may be expressed as a cell surface protein or be secreted. Expression of the N-Cadherin or LY6-E protein/immunogen results in the generation of prophylactic or therapeutic humoral and cellular immunity against prostate cancer. Various prophylactic and therapeutic genetic immunization techniques known in the art may be used (for review, see information and references published at internet address www.genweb.com).

The invention further provides methods for inhibiting cellular activity (e.g., cell proliferation, activation, or propagation) of a cell expressing multiple N-Cadherin or LY6-E antigens on its cell surface. This method comprises reacting the immunoconjugates of the invention (e.g., a heterogeneous or homogenous mixture) with the cell so that the N-Cadherin or LY6-E antigens on the cell surface forms a complex with the immunoconjugates. The greater the number of N-Cadherin or LY6-E antigens on the cell surface, the greater the number of N-Cadherin or LY6-E -antibody complexes that can, respectively, be used. The greater the number of N-Cadherin or LY6-E -antibody complexes the greater the cellular activity that is inhibited.

A heterogeneous mixture includes N-Cadherin or LY6-E antibodies that recognize different or the same epitope, each antibody being conjugated to the same or different therapeutic agent. A homogenous mixture includes antibodies that recognize the same epitope, each antibody being conjugated to the same therapeutic agent.

The invention further provides methods for inhibiting the biological activity of N-Cadherin or LY6-E by respectively blocking N-Cadherin or LY6-E from binding its receptor. The methods comprises contacting an amount of N-Cadherin or LY6-E with an antibody or immunoconjugate of the invention under conditions that permit a N-Cadherin or LY6-E - immunoconjugate or N-Cadherin or LY6-E -antibody complex thereby, respectively, blocking N-Cadherin or LY6-E from binding its ligand and inhibiting the activity of N-Cadherin or LY6-E.

In some embodiments, the invention provides a method of treating cancer, particularly a cancer which overexpresses N-Cadherin or LY6-E, or of inhibiting the growth of a cancer cell overexpressing a N-Cadherin or LY6-E protein by treating a subject or contacting the cancer cell with an antibody or fragment thereof that recognizes and binds the N-Cadherin or LY6-E protein in an amount effective to inhibit the growth of the cancer cell. In some embodiments, the cancer cell is a prostate cancer cell or a bladder cancer cell. The contacting antibody can be a monoclonal antibody and/or a chimeric antibody. In some embodiments, the chimeric antibody comprises a human immunoglobulin constant region. In some embodiments, the antibody is a human antibody or comprises a human immunoglobulin constant region. In further embodiments, the antibody fragment comprises an Fab, F(ab)₂, or Fv. In other embodiments, the fragment comprises a recombinant protein having an antigen-binding region.

In another embodiment, the invention provides methods for treating cancer, particularly, a cancer overexpressing N-Cadherin or LY6-E or selectively inhibiting a cell expressing or overexpressing a N-Cadherin or LY6-E antigen by reacting any one or a combination of the immunoconjugates of the invention with the cell in an amount sufficient to inhibit the cell. Such amounts include an amount to kill the cell or an amount sufficient to inhibit cell growth or proliferation. As discussed supra the dose and dosage regimen will depend on the nature of the disease or disorder to be treated associated with N-Cadherin or LY6-E, its population, the site to which the antibodies are to be directed, the characteristics of the particular immunotoxin, and the patient. For example, the amount of immunoconjugate can be in the range of 0.1 to 200 mg/kg of patient weight. The immunoconjugate can comprise the anti- N-Cadherin or LY6-E antibody or the fragment linked to a therapeutic agent. The therapeutic agent can be cytotoxic agent. The cytotoxic agent can be selected from a group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria afficinalis inhibitor, maytansinoids, and glucocorticoidricin. The therapeutic agent can be a radioactive isotope. The therapeutic isotope can be selected from the group consisting of²¹²Bi, ¹³¹I, ¹¹¹In, ⁹⁰Y and ¹⁸⁶Re.

In any of the embodiments above, a chemotherapeutic drug and/or radiation therapy can be administered further. In some embodiments, the patient also receives hormone antagonist therapy. The contacting of the patient with the antibody or antibody fragment, can be by administering the antibody to the patient intravenously, intraperitoneally, intramuscularly, intratumorally, or intradermally. In some embodiments, the patient has a urogenital cancer (e.g., bladder cancer, prostate cancer). In some embodiments of the above, the patient suffers from prostate cancer and optionally further receives patient hormone ablation therapy. In some embodiments, the contacting comprises administering the antibody directly into the cancer or a metastasis of the cancer.

In some embodiments, the immunoconjugate has a cytotoxic agent which is a small molecule. Toxins such as maytansin, maytansinoids, saporin, gelonin, ricin or calicheamicin and analogs or derivatives thereof are also suitable. Other cytotoxic agents that can be conjugated to the anti- N-Cadherin or LY6-E antibodies include BCNU, streptozoicin, vincristine and 5-fluorouracil. Enzymatically active toxins and fragments thereof can also be used. The radio-effector moieties may be incorporated in the conjugate in known ways (e.g., bifunctional linkers, fusion proteins). The antibodies of the present invention may also be conjugated to an effector moiety which is an enzyme which converts a prodrug to an active chemotherapeutic agent. See, WO 88/07378; U. S. Patent No. 4,975, 278; and U.S. Patent No. 6,949,245. The antibody or immunoconjugate may optionally be linked to nonprotein polymers (e. g., polyethylene glycol, polypropylene glycol, polyoxyalkylenes, or copolymers of polyethylene glycol and polypropylene glycol).

Conjugates of the antibody and cytotoxic agent may be made using methods well known in the art (see, U.S. Patent No. 6,949,245). For instance, the conjugates may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acidlabile linker, peptidase-sensitive linker, dimethyl linker or disulfide-containing linker (Chari et al. Cancer Research 52: 127-131 (1992)) may be used.

### Methods of Administration and Formulation

The anti- N-cadherin or Ly6-E antibodies or immunoconjugates are administered to a human patient in accord with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred. The administration may be local or systemic.

The compositions for administration will commonly comprise an agent as described herein (e.g., N-cadherin and Ly6-E inhibitors, N-cadherin and Ly6-E antibodies and immunoconjugates, N-cadherin and Ly6-E siRNA and vectors thereof) dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the patient's needs.

Thus, a typical pharmaceutical composition for intravenous administration will vary according to the agent. Actual methods for preparing parenterally administrable compositions will be known or apparent to those skilled in the art and are described in more detail in such publications as Remington's Pharmaceutical Science, 15th ed., Mack Publishing Company, Easton, Pa. (1980).

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that antibodies when administered orally, should be protected from digestion. This is typically accomplished either by complexing the molecules with a composition to render them resistant to acidic and enzymatic hydrolysis, or by packaging the molecules in an appropriately resistant carrier, such as a liposome or a protection barrier. Means of protecting agents from digestion are well known in the art.

Pharmaceutical formulations, particularly, of the antibodies and immunoconjugates and inhibitors for use with the present invention can be prepared by mixing an antibody having the desired degree of purity with optional pharmaceutically acceptable carriers, excipients or stabilizers. Such formulations can be lyophilized formulations or aqueous solutions. Acceptable carriers, excipients, or stabilizers are nontoxic to recipients at the dosages and concentrations used. Acceptable carriers, excipients or stabilizers can be acetate, phosphate, citrate, and other organic acids; antioxidants (e.g., ascorbic acid) preservatives low molecular weight polypeptides; proteins, such as serum albumin or gelatin, or hydrophilic polymers such as polyvinylpyllolidone; and amino acids, monosaccharides, disaccharides, and other carbohydrates including glucose, mannose, or dextrins; chelating agents; and ionic and non-ionic surfactants (e.g., polysorbate); salt-forming counter-ions such as sodium; metal complexes (e. g. Zn-protein complexes); and/or non-ionic surfactants. The antibody can be formulated at a concentration of between 0.5 - 200 mg/ml, or between 10-50 mg/ml.

The formulation may also provide additional active compounds, including, chemotherapeutic agents, cytotoxic agents, cytokines, growth inhibitory agent, and anti-hormonal agent. The active ingredients may also prepared as sustained-release preparations (e.g., semi-permeable matrices of solid hydrophobic polymers (e.g., polyesters, hydrogels (for example, poly (2-hydroxyethyl-methacrylate), or poly (vinylalcohol)), polylactides. The antibodies and immunocongugates may also be entrapped in microcapsules prepared, for example, by coacervation techniques or by interfacial polymerization, for example, hydroxymethylcellulose or gelatin microcapsules and poly- (methylmethacylate) microcapsules, respectively, in colloidal drug delivery systems (for example, liposomes, albumin microspheres, microemulsions, nano-particles and nanocapsules) or in macroemulsions.

The compositions can be administered for therapeutic or prophylactic treatments. In therapeutic applications, compositions are administered to a patient suffering from a disease (e.g., cancer) in a "therapeutically effective dose." Amounts effective for this use will depend upon the severity of the disease and the general state of the patient's health. Single or multiple administrations of the compositions may be administered depending on the dosage and frequency as required and tolerated by the patient. A "patient" or "subject" for the purposes of the present invention includes both humans and other animals, particularly mammals. Thus the methods are applicable to both human therapy and veterinary applications. In the preferred embodiment the patient is a mammal, preferably a primate, and in the most preferred embodiment the patient is human. Other known cancer therapies can be used in combination with the methods of the invention. For example, the compositions for use according to the invention may also be used to target or sensitize a cell to other cancer therapeutic agents such as 5FU, vinblastine, actinomycin D, cisplatin, methotrexate, and the like.

In other embodiments, the methods of the invention with other cancer therapies (e.g, radical prostatectomy), radiation therapy (external beam or brachytherapy), hormone therapy (e.g., orchiectomy, LHRH-analog therapy to suppress testosterone production, anti-androgen therapy), or chemotherapy. Radical prostatectomy involves removal of the entire prostate gland plus some surrounding tissue. This treatment is used commonly when the cancer is thought not to have spread beyond the tissue. Radiation therapy is commonly used to treat prostate cancer that is still confined to the prostate gland, or has spread to nearby tissue. If the disease is more advanced, radiation may be used to reduce the size of the tumor. Hormone therapy is often used for patients whose prostate cancer has spread beyond the prostate or has recurred. The objective of hormone therapy is to lower levels of the male hormones, androgens and thereby cause the prostate cancer to shrink or grow more slowly. Luteinizing hormone-releasing hormone (LHRH) agonists decrease the production of testosterone. These agents may be injected either monthly or longer. Two such analogs are leuprolide and goserelin. Anti-androgens (e.g., flutamide, bicalutamide, and nilutamide) may also be used. Total androgen blockade refers to the use of anti-androgens in combination with orchiectomy or LHRH analogs, the s combination is called. Chemotherapy is an option for patients whose prostate cancer has spread outside of the prostate gland and for whom hormone therapy has failed. It is not expected to destroy all of the cancer cells, but it may slow tumor growth and reduce pain. Some of the chemotherapy drugs used in treating prostate cancer that has returned or continued to grow and spread after treatment with hormonal therapy include doxorubicin (Adriamycin), estramustine, etoposide, mitoxantrone, vinblastine, and paclitaxel. Two or more drugs are often given together to reduce the likelihood of the cancer cells becoming resistant to chemotherapy. Small cell carcinoma is a rare type of prostate cancer that is more likely to respond to chemotherapy than to hormonal therapy.

In some embodiments, a "cardioprotectant" is also administered with the N-cadherin or Ly6-E antibody, N-cadherin or Ly6-E binding inhibitor, or N-cadherin or Ly6-E siRNA molecule for use to according to the invention (see, U.S. Patent No. 6,949,245). A cardioprotectant is a compound or composition which prevents or reduces myocardial dysfunction (i.e. cardiomyopathy and/or congestive heart failure) associated with administration of a drug, such as an anthracycline antibiotic to a patient. The cardioprotectant may, for example, block or reduce a free-radical-mediated cardiotoxic effect and/or prevent or reduce oxidative-stress injury. Examples of cardioprotectants encompassed by the present definition include the iron-chelating agent dexrazoxane (ICRF-187) (Seifert et al. The Annals of Pharmacotherapy 28:1063-1072 (1994)); a lipid-lowering agent and/or anti-oxidant such as probucol (Singal et al. J. Mol. Cell Cardiol. 27:1055-1063 (1995)); amifostine (aminothiol 2-[(3-aminopropyl)amino]ethanethiol-dihydrogen phosphate ester, also called WR-2721, and the dephosphorylated cellular uptake form thereof called WR-1065) and S-3-(3-methylaminopropylamino)propylphosphoro- thioic acid (WR-151327), see Green et al. Cancer Research 54:738-741 (1994); digoxin (Bristow, M. R. In: Bristow M R, ed. Drug-Induced Heart Disease. New York: Elsevier 191-215 (1980)); beta-blockers such as metoprolol (Hjalmarson et al. Drugs 47:Suppl 4:31-9 (1994); and Shaddy et al. Am. Heart J. 129:197-9 (1995)); vitamin E; ascorbic acid (vitamin C); free radical scavengers such as oleanolic acid, ursolic acid and N-acetylcysteine (NAC); spin trapping compounds such as alpha-phenyl-tert-butyl nitrone (PBN); (Paracchini et al., Anticancer Res. 13:1607-1612 (1993)); selenoorganic compounds such as P251 (Elbesen); and the like.

The combined administrations contemplates coadministration, using separate formulations or a single pharmaceutical formulation, and consecutive administration in either order, wherein preferably there is a time period while both (or all) active agents simultaneously exert their biological activities.

Molecules and compounds identified that indirectly or directly modulate the expression and/or function of a N-cadherin or Ly6-E protein can be useful in treating cancers that,respectively, overexpress N-cadherin or Ly6-E. N-cadherin or Ly6-E protein modulators can be administered alone or co-administered in combination with conventional chemotherapy, radiotherapy or immunotherapy as well as currently developed therapeutics.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, e.g., sucrose, as well as pastilles comprising the active ingredient in an inert base, such as gelatin and glycerin or sucrose and acacia emulsions, gels, and the like containing, in addition to the active ingredient, carriers known in the art.

The compound of choice, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane, nitrogen, and the like.

Suitable formulations for rectal administration include, for example, suppositories, which consist of the packaged nucleic acid with a suppository base. Suitable suppository bases include natural or synthetic triglycerides or paraffin hydrocarbons. In addition, it is also possible to use gelatin rectal capsules which consist of a combination of the compound of choice with a base, including, for example, liquid triglycerides, polyethylene glycols, and paraffin hydrocarbons.

Formulations suitable for parenteral administration, such as, for example, by intraarticular (in the joints), intravenous, intramuscular, intratumoral, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. Parenteral administration, oral administration, and intravenous administration are the preferred methods of administration. The formulations of compounds can be presented in unit-dose or multi-dose sealed containers, such as ampules and vials.

Injection solutions and suspensions can be prepared from sterile powders, granules, and tablets of the kind previously described. Cells transduced by nucleic acids for *ex vivo* therapy can also be administered intravenously or parenterally as described above.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form. The composition can, if desired, also contain other compatible therapeutic agents.

Preferred pharmaceutical preparations deliver one or more active N-cadherin or Ly6-E protein modulators, optionally in combination with one or more chemotherapeutic agents or immunotherapeutic agents, in a sustained release formulation. Typically, the N-cadherin or Ly6-E modulator is administered therapeutically as a sensitizing agent that increases the susceptibility of tumor cells to other cytotoxic cancer therapies, including chemotherapy, radiation therapy, immunotherapy and hormonal therapy.

In therapeutic use for the treatment of cancer, the N-cadherin or Ly6-E modulators or inhibitors utilized in the pharmaceutical method of the invention are administered at the initial dosage of about 0.001 mg/kg to about 1000 mg/kg daily. A daily dose range of about 0.01 mg/kg to about 500 mg/kg, or about 0.1 mg/kg to about 200 mg/kg, or about 1 mg/kg to about 100 mg/kg, or about 10 mg/kg to about 50 mg/kg, can be used. The dosages, however, may be varied depending upon the requirements of the patient, the severity of the condition being treated, and the compound being employed. For example, dosages can be empirically determined considering the type and stage of cancer diagnosed in a particular patient. The dose administered to a patient, in the context of the present invention should be sufficient to effect a beneficial therapeutic response in the patient over time. The size of the dose also will be determined by the existence, nature, and extent of any adverse side-effects that accompany the administration of a particular vector, or transduced cell type in a particular patient. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. For convenience, the total daily dosage may be divided and administered in portions during the day, if desired.

The pharmaceutical preparations (e.g., N-cadherin or Ly6-E siRNAs, N-cadherin or Ly6-E antibodies, N-cadherin or Ly6-E vaccines, N-cadherin or Ly6-E inhibitors, and immunoconjudates) for use according to the invention are typically delivered to a mammal, including humans and non-human mammals. Non-human mammals treated using the present methods include domesticated animals (i.e., canine, feline, murine, rodentia, and lagomorpha) and agricultural animals (bovine, equine, ovine, porcine).

### Assays for modulators of N-Cadherin or LY6-E protein

Modulation of a N-Cadherin or LY6-E protein, and corresponding modulation of cellular, e.g., tumor cell, proliferation, can be assessed using a variety *of in vitro* and *in vivo* assays, including cell-based models. Such assays can be used to test for inhibitors and activators of a N-Cadherin or LY6-E protein, and, consequently, inhibitors and activators of cellular proliferation, including modulators of chemotherapeutic sensitivity and toxicity. Such modulators of a N-Cadherin or LY6-E protein are useful for treating disorders related to pathological cell proliferation, e.g., cancer. Modulators of N-Cadherin or LY6-E protein are tested using either recombinant or naturally occurring N-Cadherin or LY6-E, preferably human N-Cadherin or LY6-E.

Measurement of cellular proliferation modulation with a N-Cadherin or LY6-E protein or a cell expressing a N-Cadherin or LY6-E protein, either recombinant or naturally occurring, can be performed using a variety of assays, *in vitro, in vivo,* and ex *vivo,* as described herein. A suitable physical, chemical or phenotypic change that affects activity, e.g., enzymatic activity such as kinase activity, cell proliferation, or ligand binding (e.g., a N-Cadherin or LY6-E protein receptor) can be used to assess the influence of a test compound on the polypeptide of this invention. When the functional effects are determined using intact cells or animals, one can also measure a variety of effects, such as, ligand binding, kinase activity, transcriptional changes to both known and uncharacterized genetic markers (e.g., northern blots), changes in cell metabolism, changes related to cellular proliferation, cell surface marker expression, DNA synthesis, marker and dye dilution assays (e.g., GFP and cell tracker assays), contact inhibition, tumor growth in nude mice, etc.

### In vitro assays

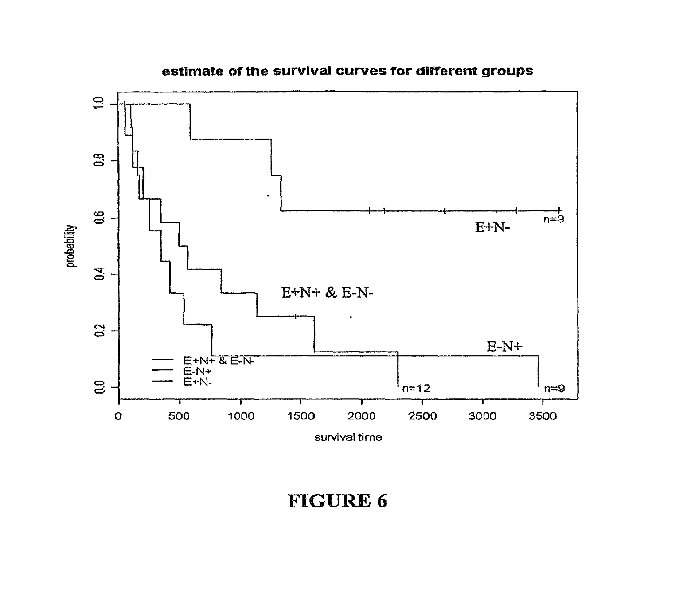

Assays to identify compounds with N-Cadherin or LY6-E modulating activity can be performed *in vitro.* Such assays can use a full length N-Cadherin or LY6-E protein or a variant thereof *(see, e.g.,* Figures6 and 7, respectively), or a mutant thereof, or a fragment of a N-Cadherin or LY6-E protein. Purified recombinant or naturally occurring N-Cadherin or LY6-E protein can be used in the *in vitro* methods of the invention. In addition to purified N-Cadherin or LY6-E protein, the recombinant or naturally occurring N-Cadherin or LY6-E protein can be part of a cellular lysate or a cell membrane. As described below, the binding assay can be either solid state or soluble. Preferably, the protein or membrane is bound to a solid support, either covalently or non-covalently. Often, the *in vitro* assays of the invention are substrate or ligand binding or affinity assays, either non-competitive or competitive. Other *in vitro* assays include measuring changes in spectroscopic (e.g., fluorescence, absorbance, refractive index), hydrodynamic (e.g., shape), chromatographic, or solubility properties for the protein. Other *in vitro* assays include enzymatic activity assays, such as phosphorylation or autophosphorylation assays).

In one embodiment, a high throughput binding assay is performed in which the N-Cadherin or LY6-E protein or a fragment thereof is contacted with a potential modulator and incubated for a suitable amount of time. In one embodiment, the potential modulator is bound to a solid support, and the N-Cadherin or LY6-E protein is added. In another embodiment, the N-Cadherin or LY6-E protein is bound to a solid support. A wide variety of modulators can be used, as described below, including small organic molecules, peptides, antibodies, and N-Cadherin or LY6-E ligand analogs. A wide variety of assays can be used to identify N-Cadherin or LY6-E -modulator binding, including labeled protein-protein binding assays, electrophoretic mobility shifts, immunoassays, enzymatic assays such as kinase assays, and the like. In some cases, the binding of the candidate modulator is determined through the use of competitive binding assays, where interference with binding of a known ligand or substrate is measured in the presence of a potential modulator.

In one embodiment, microtiter plates are first coated with either a N-Cadherin or LY6-E protein or a N-Cadherin or LY6-E protein receptor, and then exposed to one or more test compounds potentially capable of inhibiting the binding of a N-Cadherin or LY6-E protein to a N-Cadherin or LY6-E protein receptor. A labeled (*i.e.,* fluorescent, enzymatic, radioactive isotope) binding partner of the coated protein, either a N-Cadherin or LY6-E protein receptor or a N-Cadherin or LY6-E protein, is then exposed to the coated protein and test compounds. Unbound protein is washed away as necessary in between exposures to a N-Cadherin or LY6-E protein, a N-Cadherin or LY6-E protein receptor, or a test compound. An absence of detectable signal indicates that the test compound inhibited the binding interaction between a N-Cadherin or LY6-E protein and, respectively, a N-Cadherin or LY6-E protein receptor. The presence of detectable signal *(i.e.,* fluorescence, colorimetric, radioactivity) indicates that the test compound did not inhibit the binding interaction between a N-Cadherin or LY6-E protein and, respectively, a N-Cadherin or LY6-E protein receptor. The presence or absence of detectable signal is compared to a control sample that was not exposed to a test compound, which exhibits uninhibited signal. In some embodiments the binding partner is unlabeled, but exposed to a labeled antibody that specifically binds the binding partner.

### Cell-based in vivo assays

In another embodiment, N-Cadherin or LY6-E protein is expressed in a cell, and functional, e.g., physical and chemical or phenotypic, changes are assayed to identify N-Cadherin or LY6-E and modulators of cellular proliferation, e.g., tumor cell proliferation. Cells expressing N-Cadherin or LY6-E proteins can also be used in binding assays and enzymatic assays. Any suitable functional effect can be measured, as described herein. For example, cellular morphology (e.g., cell volume, nuclear volume, cell perimeter, and nuclear perimeter), ligand binding, kinase activity, apoptosis, cell surface marker expression, cellular proliferation, GFP positivity and dye dilution assays (e.g., cell tracker assays with dyes that bind to cell membranes), DNA synthesis assays (e.g., ³H-thymidine and fluorescent DNA-binding dyes such as BrdU or Hoechst dye with FACS analysis), are all suitable assays to identify potential modulators using a cell based system. Suitable cells for such cell based assays include both primary cancer or tumor cells and cell lines, as described herein, e.g., A549 (lung), MCF7 (breast, p53 wild-type), H1299 (lung, p53 null), Hela (cervical), PC3 (prostate, p53 mutant), MDA-MB-231 (breast, p53 wild-type). Cancer cell lines can be p53 mutant, p53 null, or express wild type p53. The N-Cadherin or LY6-E protein can be naturally occurring or recombinant. Also, fragments of N-Cadherin or LY6-E or chimeric N-Cadherin or LY6-E proteins can be used in cell based assays.

Cellular N-Cadherin or LY6-E polypeptide levels can be determined by measuring the level of protein or mRNA. The level of N-Cadherin or LY6-E protein or proteins related to N-Cadherin or LY6-E are measured using immunoassays such as western blotting ELISA and the like with an antibody that selectively binds, respectively, to the N-Cadherin or LY6-E polypeptide or a fragment thereof. For measurement of mRNA, amplification, e.g., using PCR, LCR, or hybridization assays, e.g., northern hybridization, RNAse protection, dot blotting, are preferred. The level of protein or mRNA is detected using directly or indirectly labeled detection agents, e.g., fluorescently or radioactively labeled nucleic acids, radioactively or enzymatically labeled antibodies, and the like, as described herein.

Alternatively, N-Cadherin or LY6-E expression can be measured using a reporter gene system. Such a system can be devised using an N-Cadherin or LY6-E protein promoter operably linked to a reporter gene such as chloramphenicol acetyltransferase, firefly luciferase, bacterial luciferase, β-galactosidase and alkaline phosphatase. Furthermore, the protein of interest can be used as an indirect reporter via attachment to a second reporter such as red or green fluorescent protein *(see, e.g.,* Mistili & Spector, Nature Biotechnology 15:961-964 (1997)). The reporter construct is typically transfected into a cell. After treatment with a potential modulator, the amount of reporter gene transcription, translation, or activity is measured according to standard techniques known to those of skill in the art.

### Animal models

Animal models of cellular proliferation also find use in screening for modulators of cellular proliferation. Similarly, transgenic animal technology including gene knockout technology, for example as a result of homologous recombination with an appropriate gene targeting vector, or gene overexpression, will result in the absence or increased expression of the N-Cadherin or LY6-E protein. The same technology can also be applied to make knock-out cells. When desired, tissue-specific expression or knockout of the N-Cadherin or LY6-E protein may be necessary. Transgenic animals generated by such methods find use as animal models of cellular proliferation and are additionally useful in screening for modulators of cellular proliferation.

Knock-out cells and transgenic mice can be made by insertion of a marker gene or other heterologous gene into an endogenous N-Cadherin or LY6-E gene site in the mouse genome via homologous recombination. Such mice can also be made by substituting an endogenous N-Cadherin or LY6-E, respectively, with a mutated version of the N-Cadherin or LY6-E gene, or by, respectively, mutating an endogenous N-Cadherin or LY6-E, e.g., by exposure to carcinogens.

A DNA construct is introduced into the nuclei of embryonic stem cells. Cells containing the newly engineered genetic lesion are injected into a host mouse embryo, which is re-implanted into a recipient female. Some of these embryos develop into chimeric mice that possess germ cells partially derived from the mutant cell line. Therefore, by breeding the chimeric mice it is possible to obtain a new line of mice containing the introduced genetic lesion (see, *e.g.,* Capecchi et al., Science 244:1288 (1989)). Chimeric targeted mice can be derived according to Hogan et al., Manipulating the Mouse Embryo: A Laboratory Manual, Cold Spring Harbor Laboratory (1988), Teratocarcinomas and Embryonic Stem Cells: A Practical Approach, Robertson, ed., IRL Press, Washington, D.C., (1987), and Pinkert, Transgenic Animal Technology: A Laboratory Handbook, Academic Press (2003).

### Exemplary assays

### Soft agar growth or colony formation in suspension

Normal cells require a solid substrate to attach and grow. When the cells are transformed, they lose this phenotype and grow detached from the substrate. For example, transformed cells can grow in stirred suspension culture or suspended in semi-solid media, such as semi-solid or soft agar. The transformed cells, when transfected with tumor suppressor genes, regenerate normal phenotype and require a solid substrate to attach and grow.

Soft agar growth or colony formation in suspension assays can be used to identify N-Cadherin or LY6-E modulators. Typically, transformed host cells (e.g., cells that grow on soft agar) are used in this assay. For example, RKO or HCT116 cell lines can be used. Techniques for soft agar growth or colony formation in suspension assays are described in Freshney, Culture of Animal Cells a Manual of Basic Technique, 3rd ed., Wiley-Liss, New York (1994), herein incorporated by reference. *See also,* the methods section of Garkavtsev *et al.* (1996), *supra,* herein incorporated by reference.

### Contact inhibition and density limitation of growth

Normal cells typically grow in a flat and organized pattern in a petri dish until they touch other cells. When the cells touch one another, they are contact inhibited and stop growing. When cells are transformed, however, the cells are not contact inhibited and continue to grow to high densities in disorganized foci. Thus, the transformed cells grow to a higher saturation density than normal cells. This can be detected morphologically by the formation of a disoriented monolayer of cells or rounded cells in foci within the regular pattern of normal surrounding cells. Alternatively, labeling index with [³H]-thymidine at saturation density can be used to measure density limitation of growth. *See* Freshney (1994), *supra.* The transformed cells, when contacted with cellular proliferation modulators, regenerate a normal phenotype and become contact inhibited and would grow to a lower density.

Contact inhibition and density limitation of growth assays can be used to identify N-Cadherin or LY6-E modulators which are capable of inhibiting abnormal proliferation and transformation in host cells. Typically, transformed host cells (e.g., cells that are not contact inhibited) are used in this assay. For example, RKO or HCT116 cell lines can be used. In this assay, labeling index with [³H]-thymidine at saturation density is a preferred method of measuring density limitation of growth. Transformed host cells are contacted with a potential N-cadherin or Ly6-E modulator and are grown for 24 hours at saturation density in nonlimiting medium conditions. The percentage of cells labeling with [³H]-thymidine is determined autoradiographically. *See,* Freshney (1994), *supra.* The host cells contacted with a N-cadherin or Ly6-E modulator would give arise to a lower labeling index compared to control (e.g., transformed host cells transfected with a vector lacking an insert).

### Growth factor or serum dependence

Growth factor or serum dependence can be used as an assay to identify N-cadherin or Ly6-E modulators. Transformed cells have a lower serum dependence than their normal counterparts *(see, e.g.,* Temin, J. Natl. Cancer Insti. 37:167-175 (1966); Eagle et al., J. Exp. Med. 131:836-879 (1970)); Freshney, *supra.* This is in part due to release of various growth factors by the transformed cells. When transformed cells are contacted with a N-Cadherin or LY6-E modulator, the cells would reacquire serum dependence and would release growth factors at a lower level.

### Tumor specific markers levels

Tumor cells release an increased amount of certain factors (hereinafter "tumor specific markers") than their normal counterparts. For example, plasminogen activator (PA) is released from human glioma at a higher level than from normal brain cells *(see, e.g.,* Gullino, Angiogenesis, tumor vascularization, and potential interference with tumor growth. In Mihich (ed.): "Biological Responses in Cancer." New York, Academic Press, pp. 178-184 (1985)). Similarly, tumor angiogenesis factor (TAF) is released at a higher level in tumor cells than their normal counterparts. *See, e.g.,* Folkman, Angiogenesis and cancer, Sem Cancer Biol. (1992*)).*

Tumor specific markers can be assayed to identify N-Cadherin or LY6-E modulators which decrease the level of release of these markers from host cells. Typically, transformed or tumorigenic host cells are used. Various techniques which measure the release of these factors are described in Freshney (1994), *supra.* Also, *see,* Unkless et al. , J. Biol. Chem. 249:4295-4305 (1974); Strickland & Beers, J. Biol. Chem. 251:5694-5702 (1976); Whur et al., Br. J. Cancer 42:305-312 (1980); Gulino, Angiogenesis, tumor vascularization, and potential interference with tumor growth. In Mihich, E. (ed): "Biological Responses in Cancer." New York, Plenum (1985); Freshney Anticancer Res. 5:111-130 (1985).

### Invasiveness into Matrigel

The degree of invasiveness into Matrigel or some other extracellular matrix constituent can be used as an assay to identify N-Cadherin or LY6-E modulators which are capable of inhibiting abnormal cell proliferation and tumor growth. Tumor cells exhibit a good correlation between malignancy and invasiveness of cells into Matrigel or some other extracellular matrix constituent. In this assay, tumorigenic cells are typically used as host cells. Therefore, N-Cadherin or LY6-E modulators can be identified by measuring changes in the level of invasiveness between the host cells before and after the introduction of potential modulators. If a compound modulates N-Cadherin or LY6-E, its expression in tumorigenic host cells would affect invasiveness.

Techniques described in Freshney (1994), *supra,* can be used. Briefly, the level of invasion of host cells can be measured by using filters coated with Matrigel or some other extracellular matrix constituent. Penetration into the gel, or through to the distal side of the filter, is rated as invasiveness, and rated histologically by number of cells and distance moved, or by prelabeling the cells with ¹²⁵I and counting the radioactivity on the distal side of the filter or bottom of the dish. *See, e.g.,* Freshney (1984), *supra.*

### Tumor growth in vivo

Effects of N-Cadherin or LY6-E modulators on cell growth can be tested in transgenic or immune-suppressed mice. Knock-out transgenic mice can be made, in which the endogenous N-Cadherin or LY6-E gene is disrupted. Such knock-out mice can be used to study effects ofN-Cadherin or LY6-E, e.g., as a cancer model, as a means of assaying *in vivo* for compounds that modulate N-Cadherin or LY6-E, and to test the effects of restoring a wild-type or mutant N-Cadherin or LY6-E to a knock-out mouse.

Knock-out cells and transgenic mice can be made by insertion of a marker gene or other heterologous gene into the endogenous N-Cadherin or LY6-E gene site in the mouse genome via homologous recombination. Such mice can also be made by substituting the endogenous N-Cadherin or LY6-E with a mutated version ofN-Cadherin or LY6-E, or by mutating the endogenous N-Cadherin or LY6-E, e.g., by exposure to carcinogens.

A DNA construct is introduced into the nuclei of embryonic stem cells. Cells containing the newly engineered genetic lesion are injected into a host mouse embryo, which is re-implanted into a recipient female. Some of these embryos develop into chimeric mice that possess germ cells partially derived from the mutant cell line. Therefore, by breeding the chimeric mice it is possible to obtain a new line of mice containing the introduced genetic lesion *(see, e.g.,* Capecchi et al., Science 244:1288 (1989)). Chimeric targeted mice can be derived according to Hogan et al., Manipulating the Mouse Embryo: A Laboratory Manual, Cold Spring Harbor Laboratory (1988) and Teratocarcinomas and Embryonic Stem Cells: A Practical Approach, Robertson, ed., IRL Press, Washington, D.C., (1987). These knock-out mice can be used as hosts to test the effects of various N-Cadherin or LY6-E modulators on cell growth.

Alternatively, various immune-suppressed or immune-deficient host animals can be used. For example, genetically athymic "nude" mouse *(see, e.g.,* Giovanella et al., J. Natl. Cancer Inst. 52:921 (1974)), a SCID mouse, a thymectomized mouse, or an irradiated mouse *(see, e.g.,* Bradley et al., Br. J. Cancer 38:263 (1978); Selby et al., Br. J. Cancer 41:52 (1980)) can be used as a host. Transplantable tumor cells (typically about 10⁶ cells) injected into isogenic hosts will produce invasive tumors in a high proportions of cases, while normal cells of similar origin will not. Hosts are treated with N-Cadherin or LY6-E modulators, e.g., by injection. After a suitable length of time, preferably 4-8 weeks, tumor growth is measured (e.g., by volume or by its two largest dimensions) and compared to the control. Tumors that have statistically significant reduction (using, e.g., Student's T test) are said to have inhibited growth. Using reduction of tumor size as an assay, N-Cadherin or LY6-E modulators which are capable, e.g., of inhibiting abnormal cell proliferation can be identified.

### Screening Methods

The present invention also provides methods of identifying compounds that inhibit the binding of a N-Cadherin or LY6-E protein, respectively, to a N-Cadherin or LY6-E receptor, wherein said compounds find use in inhibiting the growth of and promoting the regression of a tumor that overexpresses N-Cadherin or LY6-E protein, for example a urogenital cancer tumor, including a prostate or bladder cancer tumor.

Using the assays described herein, one can identify lead compounds that are suitable for further testing to identify those that are therapeutically effective modulating agents by screening a variety of compounds and mixtures of compounds for their ability to decrease, inhibit the binding of a N-Cadherin or LY6-E protein, respectively, to a N-Cadherin or LY6-E receptor. Compounds of interest can be either synthetic or naturally occurring.

Screening assays can be carried out *in vitro or in vivo.* Typically, initial screening assays are carried out *in vitro,* and can be confirmed *in vivo* using cell based assays or animal models. For instance, proteins of the regenerating gene family are involved with cell proliferation. Therefore, compounds that inhibit the binding of a N-Cadherin or LY6-E protein, respectively, to a N-Cadherin or LY6-E receptor can inhibit cell proliferation resulting from this binding interaction in comparison to cells unexposed to a test compound. Also, the binding of a N-Cadherin or LY6-E protein, respectively, to a N-Cadherin or LY6-E receptor is involved with tissue injury responses, inflammation, and dysplasia. In animal models, compounds that inhibit the binding of a N-Cadherin or LY6-E protein, respectively, to its receptor can, for example, inhibit wound healing or the progression of dysplasia in comparison to an animal unexposed to a test compound. *See, for example,* Zhang, et al., World J Gastroenter (2003) 9:2635-41.

Usually a compound that inhibits the binding of N-Cadherin or LY6-E, respectively, to a N-cadherin or Ly6-E receptor is synthetic. The screening methods are designed to screen large chemical libraries by automating the assay steps and providing compounds from any convenient source to assays, which are typically run in parallel (e.g., in microtiter formats on microtiter plates in robotic assays).

The invention provides *in vitro* assays for inhibiting N-Cadherin or LY6-E binding to its receptor in a high throughput format. For each of the assay formats described, "no modulator" control reactions which do not include a modulator provide a background level of N-Cadherin or LY6-E binding interaction to its receptor or receptors. In the high throughput assays of the invention, it is possible to screen up to several thousand different modulators in a single day. In particular, each well of a microtiter plate can be used to run a separate assay against a selected potential modulator, or, if concentration or incubation time effects are to be observed, every 5-10 wells can test a single modulator. Thus, a single standard microtiter plate can assay about 100 (96) modulators. If 1536 well plates are used, then a single plate can easily assay from about 100- about 1500 different compounds. It is possible to assay many different plates per day; assay screens for up to about 6,000-20,000, and even up to about 100,000-1,000,000 different compounds is possible using the integrated systems of the invention. The steps of labeling, addition of reagents, fluid changes, and detection are compatible with full automation, for instance using programmable robotic systems or "integrated systems" commercially available, for example, through BioTX Automation, Conroe, TX; Qiagen, Valencia, CA; Beckman Coulter, Fullerton, CA; and Caliper Life Sciences, Hopkinton, MA.

Essentially any chemical compound can be tested as a potential inhibitor of N-Cadherin or LY6-E binding to its receptor for use in the methods of the invention. Most preferred are generally compounds that can be dissolved in aqueous or organic (especially DMSO-based) solutions are used. It will be appreciated that there are many suppliers of chemical compounds, including Sigma (St. Louis, MO), Aldrich (St. Louis, MO), Sigma-Aldrich (St. Louis, MO), Fluka Chemika-Biochemica Analytika (Buchs Switzerland), as well as providers of small organic molecule and peptide libraries ready for screening, including Chembridge Corp. (San Diego, CA), Discovery Partners International (San Diego, CA), Triad Therapeutics (San Diego, CA), Nanosyn (Menlo Park, CA), Affymax (Palo Alto, CA), ComGenex (South San Francisco, CA), and Tripos, Inc. (St. Louis, MO).

In one preferred embodiment, inhibitors of the N-Cadherin or LY6-E receptor binding interaction are identified by screening a combinatorial library containing a large number of potential therapeutic compounds (potential modulator compounds). Such "combinatorial chemical or peptide libraries" can be screened in one or more assays, as described herein, to identify those library members (particular chemical species or subclasses) that display a desired characteristic activity. The compounds thus identified can serve as conventional "lead compounds" or can themselves be used as potential or actual therapeutics.

A combinatorial chemical library is a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length *(i.e.,* the number of amino acids in a polypeptide compound). Millions of chemical compounds can be synthesized through such combinatorial mixing of chemical building blocks.

Preparation and screening of combinatorial chemical libraries is well known to those of skill in the art. Such combinatorial chemical libraries include, but are not limited to, peptide libraries *(see, e.g.,* U.S. Patent 5,010,175, Furka, Int. J. Pept. Prot. Res. 37:487-493 (1991) and Houghton et al., Nature 354:84-88 (1991)). Other chemistries for generating chemical diversity libraries can also be used. Such chemistries include, but are not limited to: peptoids (PCT Publication No. WO 91/19735), encoded peptides (PCT Publication WO 93/20242), random bio-oligomers (PCT Publication No. WO 92/00091), benzodiazepines (U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with β-D-glucose scaffolding (Hirschmann et al., J. Amer. Chem. Soc. 114:9217-9218 (1992)), analogous organic syntheses of small compound libraries *(*Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries *(see,* Ausubel, Berger and Sambrook, *all supra),* peptide nucleic acid libraries *(see, e.g.,* U.S. Patent 5,539,083), antibody libraries *(see, e.g.,* Vaughn et al., Nature Biotechnology, 14(3):309-314 (1996) and PCT/US96/10287), carbohydrate libraries *(see,* e.g., Liang et al., Science, 274:1520-1522 (1996) and U.S. Patent 5,593,853), small organic molecule libraries *(see, e.g.,* benzodiazepines, Baum C&EN, Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines, 5,288,514, and the like).

Devices for the preparation of combinatorial libraries are commercially available *(see, e.g.,* 357 MPS, 390 MPS, Advanced Chem. Tech, Louisville KY, Symphony, Rainin, Woburn, MA, 433A Applied Biosystems, Foster City, CA, 9050 Plus, Millipore, Bedford, MA).

### siRNA technology

The design and making of siRNA molecules and vectors are well known to those of ordinary skill in the art. For instance, an efficient process for designing a suitable siRNA is to start at the AUG start codon of the mRNA transcript (eg., see, Figures 7, 8, 9) and scan for AA dinucleotide sequences (see, Elbashir et al. EMBO J 20: 6877-6888 (2001). Each AA and the 3' adjacent nucleotides are potential siRNA target sites. The length of the adjacent site sequence will determine the length of the siRNA. For instance, 19 adjacent sites would give a 21 Nucleotide long siRNA siRNAs with 3' overhanging UU dinucleotides are often the most effective. This approach is also compatible with using RNA pol III to transcribe hairpin siRNAs. RNA pol III terminates transcription at 4-6 nucleotide poly(T) tracts to create RNA molecules having a short poly(U) tail. However, siRNAs with other 3' terminal dinucleotide overhangs can also effectively induce RNAi and the sequence may be empirically selected. For selectivity, target sequences with more than 16-17 contiguous base pairs of homology to other coding sequences can be avoided by conducting a BLAST search (see, www.ncbi.nlm.nih.gov/BLAST.

The siRNA expression vectors to induce RNAi can have different design criteria. A vector can have inserted two inverted repeats separated by a short spacer sequence and ending with a string of T's which serve to terminate transcription. The expressed RNA transcript is predicted to fold into a short hairpin siRNA. The selection of siRNA target sequence, the length of the inverted repeats that encode the stem of a putative hairpin, the order of the inverted repeats, the length and composition of the spacer sequence that encodes the loop of the hairpin, and the presence or absence of 5'-overhangs, can vary. A preferred order of the siRNA expression cassette is sense strand, short spacer, and antisense strand. Hairp siRNAs with these various stem lengths (e.g., 15 to 30) can be suitable. The length of the loops linking sense and antisense strands of the hairpin siRNA lcan have varying lengths (e.g., 3 to 9 nucleotides, or longer). The vectors may contain promoters and expression enhancers or other regulatory elements which are operably linked to the nucleotide sequence encoding the siRNA. These control elements may be designed to allow the clinician to turn off or on the expression of the gene by adding or controlling external factors to which the regulatory elements are responsive.

In some embodiments, the invention provides a method for inhibiting the growth of a cancer cell over expressing a N-Cadherin or Ly6-E protein by contacting the cancer cell with an antibody or fragment thereof that recognized and binds the protein in an amount effective to inhibit the growth of the cancer cell. In some embodiments, the cancer cell is a prostate cancer cell or a bladder cancer cell. The contacting antibody can be a monoclonal antibody and/or a chimeric antibody. In some embodiments, the chimeric antibody comprises a human immunoglobulin constant region. In some embodiments, the antibody is a human antibody or comprises a human immunoglobulin constant region. In further embodiments, the antibody fragment comprises an Fab, F(ab)₂, or Fv. In other embodiments, the fragment comprises a recombinant protein having an antigen-binding region. In yet other embodiments, the antibody or the fragment is an immunoconjagate comprising the antibody or the fragment linked to a therapeutic agent. The therapeutic agent can be cytotoxic agent. The cytotoxic agent can be selected from a group consisting of ricin, ricin A-chain, doxorubicin, daunorubicin, taxol, ethiduim bromide, mitomycin, etoposide, tenoposide, vincristine, vinblastine, colchicine, dihydroxy anthracin dione, actinomycin D, diphteria toxin, Pseudomonas exotoxin (PE) A, PE40, abrin, arbrin A chain, modeccin A chain, alpha-sarcin, gelonin mitogellin, retstrictocin, phenomycin, enomycin, curicin, crotin, calicheamicin, sapaonaria officinalis inhibitor, maytansinoids, and glucocorticoidricin. The therapeutic agent can be a radioactive isotope. The therapeutic isotope can be selected from the group consisting of ²¹²Bi, ¹³¹I, ¹¹¹In, ⁹⁰Y and ¹⁸⁶Re. In any of the embodiments above, a chemotherapeutic drug and/or radiation therapy can be administered further. In some embodiments, the patient also receives hormone antagonist therapy. The contacting of the patient with the antibody or antibody fragment, can be by administering the antibody to the patient intravenously, intraperitoneally, intramuscularly, intratumorally, or intradermally. In some embodiments, the patient has a urogenital cancer (e.g., bladder cancer, prostate cancer). In some embodiments of the above, the patient suffers from prostate cancer and optionally further receives patient hormone ablation therapy. In some embodiments, the contacting comprises administering the antibody directly into the cancer or a metastasis of the cancer.

In some embodiments, the immunoconjugate has a cytotoxic agent which is a small molecule. Toxins such as maytansin, maytansinoids, saporin, gelonin, ricin or calicheamicin and analogs or derivatives thereof are also suitable.. Other cytotoxic agents that can be conjugated to the N-cadherin or LY6-E antibodies include BCNU, streptozoicin, vincristine and 5-fluorouracil. Enzymatically active toxins and fragments thereof can also be used. The radio-or other labels may be incorporated in the conjugate in known ways (e.g., bifunctional linkers, fusion proteins). The antibodies of the present invention may also be conjugated to an enzyme which converts a prodrug to an active chemotherapeutic agent. See, WO 88/07378 and U. S. Patent No. 4,975, 278. The antibody or immunoconjugate may optionally be linked to nonprotein polymers (e. g., polyethylene glycol, polypropylene glycol, polyoxyalkylenes, or copolymers of polyethylene glycol and polypropylene glycol).

The compositions for administration will commonly comprise an agent as described herein dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the patient's needs.

Thus, a typical pharmaceutical composition for intravenous administration may provide from about 0.1 to 100 mg per patient per day. Dosages from 0.1 up to about 100 mg per patient per day may be used. Substantially higher dosages are possible in topical administration. Actual methods for preparing parenterally administrable compositions will be known or apparent to those skilled in the art and are described in more detail in such publications as Remington's Pharmaceutical Science, 15th ed., Mack Publishing Company, Easton, Pa. (1980).

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that antibodies when administered orally, should be protected from digestion. This is typically accomplished either by complexing the molecules with a composition to render them resistant to acidic and enzymatic hydrolysis, or by packaging the molecules in an appropriately resistant carrier, such as a liposome or a protection barrier. Means of protecting agents from digestion are well known in the art.

Pharmaceutical formulations, particularly, of the antibodies and immunoconjugates and inhibitors for use with the present invention can be prepared by mixing an antibody having the desired degree of purity with optional pharmaceutically acceptable carriers, excipients or stabilizers. Such formulations can be lyophilized formulations or aqueous solutions. Acceptable carriers, excipients, or stabilizers are nontoxic to recipients at the dosages and concentrations used. Acceptable carriers, excipients or stabilizers can be acetate, phosphate, citrate, and other organic acids; antioxidants (e.g., ascorbic acid) preservatives low molecular weight polypeptides; proteins, such as serum albumin or gelatin, or hydrophilic polymers such as polyvinylpyllolidone; and amino acids, monosaccharides, disaccharides, and other carbohydrates including glucose, mannose, or dextrins; chelating agents; and ionic and non-ionic surfactants (e.g., polysorbate); salt-fonning counter-ions such as sodium; metal complexes (e. g. Zn-protein complexes); and/or non-ionic surfactants. The antibody can be formulated at a concentration of between 0.5 - 200 mg/ml, or between 10-50 mg/ml.

The formulation may also provide additional active compounds, including, chemotherapeutic agents, cytotoxic agents, cytokines, growth inhibitory agent, and anti-hormonal agent. The active ingredients may also prepared as sustained-release preparations (e.g., semi-permeable matrices of solid hydrophobic polymers (e.g., polyesters, hydrogels (for example, poly (2-hydroxyethyl-methacrylate), or poly (vinylalcohol) ), polylactides. The antibodies and immunocongugates may also be entrapped in microcapsules prepared, for example, by coacervation techniques or by interfacial polymerization, for example, hydroxymethylcellulose or gelatin microcapsules and poly- (methylmethacylate) microcapsules, respectively, in colloidal drug delivery systems (for example, liposomes, albumin microspheres, microemulsions, nano-particles and nanocapsules) or in macroemulsions.

The compositions containing the inhibitors and agents of the invention (e.g., antibodies) can be administered for therapeutic or prophylactic treatments. In therapeutic applications, compositions are administered to a patient suffering from a disease (e.g., cancer) in a "therapeutically effective dose." Amounts effective for this use will depend upon the severity of the disease and the general state of the patient's health. Single or multiple administrations of the compositions may be administered depending on the dosage and frequency as required and tolerated by the patient. A "patient" or "subject" for the purposes of the present invention includes both humans and other animals, particularly mammals. Thus the methods are applicable to both human therapy and veterinary applications. In the preferred embodiment the patient is a mammal, preferably a primate, and in the most preferred embodiment the patient is human. Other known cancer therapies can be used in combination with the methods of the invention. For example, inhibitors of Wnt signaling may also be used to target or sensitize a cell to other cancer therapeutic agents such as 5FU, vinblastine, actinomycin D, cisplatin, methotrexate, and the like. In other embodiments, the methods of the invention can be used with radiation therapy and the like.

### EXAMPLES

The following examples are offered to illustrate, but not limit the claimed invention.

### Example 1. Materials and Methods

### Cell Lines

The human bladder cancer cell lines (T24, EJ, J82, TCC Sup, 647 V, UC-14, SW780, RT 112, SD 148) were all maintained in RPMI 1640 1X medium (Cellgro) supplemented with 10% fetal bovine serum (Omega Scientific, Inc.) and 1% Penicillin-Streptomycin-Glutamine (PSG) (Invitrogen) at 37°C in a humidified 5% CO₂ atmosphere.

### Reagents and Antibodies

Mouse mAb against E- and N-cadherin were acquired from Zymed Laboratories Inc. (San Francisco). Another mouse anti-N-cadherin Ab (clone GC-4, Sigma, Saint-Louis) was used to neutralize N-cadherin function in Boyden chamber assays. Rabbit mAb against pan-Akt and pAkt (Ser 473) were purchased from Cell Signalling Technology. Mouse mAb anti-PTEN antibody was acquired from Santa Cruz Biotechnology. Polyclonal anti-Epidermal Growth Factor Receptor Phosphospecific antibody (PY¹⁰⁶⁸) was purchased from Biosource. LY294002 hydrochloride (PI3K inhibitor) was purchased from Sigma. It was dissolved as a concentrated stock solution in dimethyl sulfoxide (DMSO) and diluted at the time of experiment.

### Western blot

Confluent monolayered cells were washed with PBS at room temperature and extracted with hot lysis buffer. After sonicating the lysates for 20 s using a sonicator, the protein concentration of each sample was measured by the DC protein assay kit (BIO-RAD, Hercules, California, USA) in order to load equal amounts of protein on SDS-PAGE. Proteins were separated on a 10% polyacrylamide gel followed by electrophoretic transfer onto nitrocellulose. Immunoblotting was performed overnight at 4°C using primary antibodies (N-and E-cadherin, Pten, pAkt, pan-Akt and pEGFR). The blots were then incubated with a secondary antibody (anti-mouse or anti-rabbit) for 1 hour at room temperature. Detection was done with ECL detection reagent (Amersham).

For experiments involving the inhibition of N-cadherin function by GC-4 or PI3K by LY294002, cells were first serum-starved overnight (RPMI 1640, 0.1% BSA; 1% PSG) and then incubated for 1 h with or without GC-4 1:50 or with and without different concentrations of LY294002.

### Cell Proliferation and Viability Assay

MTT Assays were performed to determine the correct concentrations of GC-4 and LY294002 that inhibited Akt activity while preserving cell viability in the T24 cell line. 2.5 × 10³ cells were cultured in a 96-well plate in 200 µl of medium and allowed to attach for few hours. The cells were serum-starved overnight. The following day GC-4 (dilution 1:50) or LY294002 (10 µmol/l) was added to each well and cells were incubated at 37°C for 1 hour. 10 µl of 5-mg/ml solution of 3-(4,5-dimethylthiazol-2-yl)-2,5-diphenyltetrazolium bromide (MTT) in PBS were added to each well and the incubation was resumed for an additional 5 hr. The medium was aspirated and 200 µl DMSO was added to each well. The plates were agitated for 1 min on a shaker and the absorbance was measured at 550 nm using an ELISA plate reader.

### Invasion assays

The invasive behaviour of each cell line was measured using Matrigel-coated Boyden chambers (24 well-insert, 8 µm pore size; BD Bioscience, Bedford, MA, USA). Cells (2.5 × 10⁴ cells) were washed, resuspended in starved medium (RPMI 1640, 0.1% BSA, 1% PSG) and placed into the upper chamber. RPMI 1640 with FBS 10% was used as chemoattractant and placed in the lower chamber. The cells were incubated for 21 hours at 37°C and those that passed through the matrigel were fixed in 2% paraformaldehyde followed by staining with crystal violet 0.1%. Cells that did not pass through matrigel were removed from the insert with a cotton swab. In blocking experiments involving GC-4 (1:50), mouse mIgG1 (Sigma) was used as a control. Cells were starved overnight and seeded after one hour with GC-4 or mouse IgG1.

For experiments involving LY294002 (10 mM), cells were starved overnight and incubated also for one hour prior to seeding in the chamber. After incubation, the number of cells was evaluated by counting four independent fields of view under the microscope (10 × magnification) and results were expressed as averages with the standard error. All assays were performed in triplicate.

### In vitro invasion assay

Initial Boyden chamber invasion assays were correlated with a 3-dimensional model of bladder cancer invasion using de-epithelialized mouse or rat bladder in order to accurately reproduce the interactions between tumour cells and extra-cellular matrix. Mouse and rat bladders were obtained through a small laparotomy incision and the urothelium was removed by dissecting forceps without enzymatic digestion. The bladders were harvested in two parts and placed onto a 30 mm collagen-coated insert with the de-epithelialized surface facing upwards. After incubation for 30 minutes at 37°C, human bladder cancer cells (5 × 10⁵ cells) were washed, resuspended in 2 ml of RPMI medium and placed onto the stroma. An additional 6 ml of RPMI medium was added to the culture dish outside the culture insert in order to create an air-liquid interface within each culture insert. At 24 hours, the medium was removed from the insert. The 6 ml of medium in the culture dish was changed every 3 days. The culture was stopped at 7 and 15 days. Each bladder sample was fixed in 10% formalin for at least 12 hours and then embedded in paraffin. Histological section was done in the center of each explant and stained with hematoxylin for evaluation. The evidence of stromal invasion by cancer cells was viewed under the microscope (magnification × 40). All experiments were repeated 3 times.

### N- and E- cadherin expression in high risk Ta and T1 human bladder cancer

We analysed 12 snap-frozen non-invasive bladder carcinomas (1 pTa; 11 pT₁) and 5 snap-frozen invasive bladder carcinoma (3 pT₃; 2 pT₄). Patients gave written informed consent. The tumours were graded according to the WHO classification of 1973 and stage was determined according to the TNM classification guidelines. All tumours samples were obtained from transurethral resection or radical cystectomy without previous treatment between 1988 and 1997 (Henri Mondor Hospital, Créteil, France). A fragment was fixed for histological control and the other part were carefully collected in tubes and snap-frozen in liquid nitrogen and stored at -80°C for protein extraction. Tissue samples were lysed with RIPA lysis buffer completed with antiproteases and antiphospholipases. Proteins were extracted and the BCA kit determined the total protein concentration. Western blot analysis of these specimens was done as previously.

### N- and E- cadherin expression in invasive human bladder cancers

### Patients

A cohort of 30 patients with invasive bladder cancer treated by radical cystectomy was studied. All patients were followed from the date of surgery, at which point the sample was taken, to the date of death. Some patients died from other causes unrelated to their tumor. Other patients were lost to follow-up. These patients were censored in the survival analysis (7 cases).

### DNA array data

The DNA microarrays used in this study were the Affymetrix Human Genome U95 set, consisting of two GeneChip arrays (U95A and U95Av2), and containing almost 12600 probe sets. Each probe set consisted of 22 different oligonucleotides (11 of which are a perfect match with the target transcript and 11 of which harbor a one-nucleotide mismatch in the middle). These 22 oligonucleotides were used to measure the level of a given transcript. Chips were scanned and the intensities for each probe set were calculated using Affymetrix MAS5.0 default settings. We only kept probe sets that had the attribute "present" in at least 5% of the 30 arrays (almost 8900 probe sets). For each gene "X", patients were classified into two groups: those who had an expression measure greater than the median for all measures of gene "X", and those who had an expression measure lower than the median. This enabled us to define the two groups, respectively, as "X"+ and "X"-.

### RT-PCR

Total RNA was extracted by caesium chloride centrifugation. It was used as a template for first-strand cDNA synthesis by random priming, as previously described [23, 24]. The amount ofN-cadherin mRNA was determined by semi-quantitative radioactive RT-PCR, using TBP (TATA-binding protein) as an internal control. The primers used were GCTGGACCATTTGCTTTTGAT and GATGGGAACTTCATAGATACC for N-cadherin, AGTGAAGAACAGTCCAGACTG and CCAGGAAATAACTCTGGCTCAT for TBP. The number of cycles was selected so as to be in the exponential part of the PCR reaction (25 cycles). The PCR products were subjected to electrophoresis in 8% polyacrylamide gels. Signals were quantified with a Molecular Dynamics 300 Phosphorlmager (Molecular Dynamics, Sunnyvale, CA).

### Statistical Analysis

Statistical analysis was carried out using R software for Windows. Survival curves were estimated using the Kaplan-Meier method. A log-rank test was performed to test the null-hypothesis of there being no difference in survival between two groups. A p-value lower than 0.05 was considered statistically significant. We used the Spearman's correlation coefficient to correlate the different measurements. A Student's test was performed to test the significance of the correlation. We used logged Affymetrix data except with the correlation study where the data were unlogged.

### Example 2. N-Cadherin expression in bladder cancer cell lines is associated with Akt activation, loss of E-Cadherin, and invasive behavior.

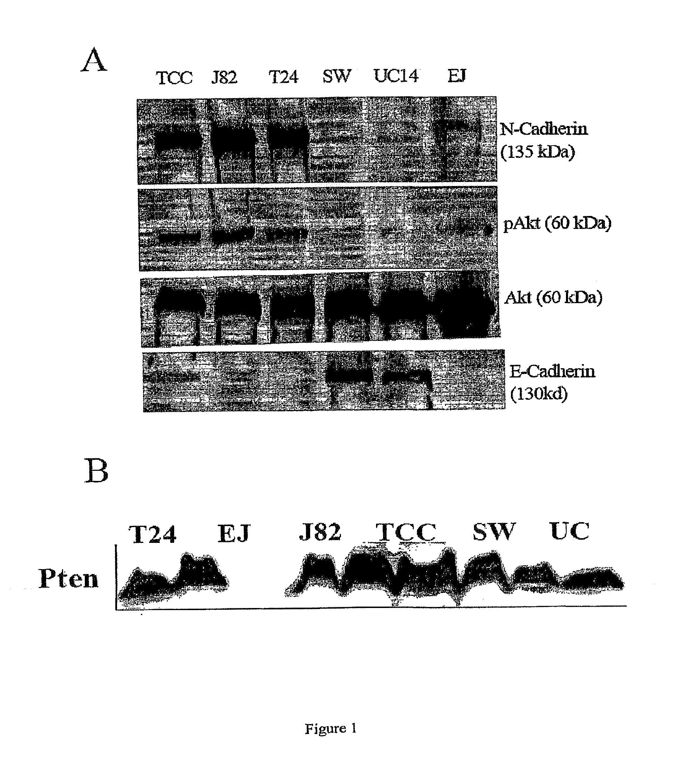

To study the possible role of EMT in bladder cancer, we first screened a panel of bladder cancer cell lines for N-Cadherin and E-Cadherin expression. As shown in Figure 1A, N-Cadherin is expressed in four of six cell lines (TCC, EJ, J82 and T24) and absent in two of six (UC14 and SW780). There is a strong inverse relationship between N-Cadherin and E-Cadherin expression, consistent with previous reports of a cadherin "switch" in cells that have undergone an EMT. UC14 and SW 780 express the highest levels of E-Cadherin and is N-Cadherin negative, while one N-cadherin positive line (TCC) retains low level expression of E-Cadherin. Based on a recent study suggesting that N-cadherin can activate Akt, we also assayed the cells for phospho- and total Akt levels. There is a marked association between N-Cadherin expression and Akt activation, with all N-Cadherin positive cell lines expressing significant levels of phospho-Akt. N-Cadherin negative cells, in contrast, express low levels of activated Akt.

To determine if Akt activation is caused by PTEN loss or mutation, we sequenced PTEN in all lines and measured PTEN protein levels (Figure 1B). A PTEN mutation was found in T24, consistent with previous reports [25]. J82 does not express detectable PTEN protein. EJ and TCC express wild-type PTEN, suggesting that Akt activation is independent of PTEN in 2/4 N-cadherin positive cell lines (data not shown). Morphologically, J82 and TCC had a fibroblastic appearance in culture, while T24 and EJ had a small, poorly differentiated form. SW780 and UC14 have an epithelial morphology.

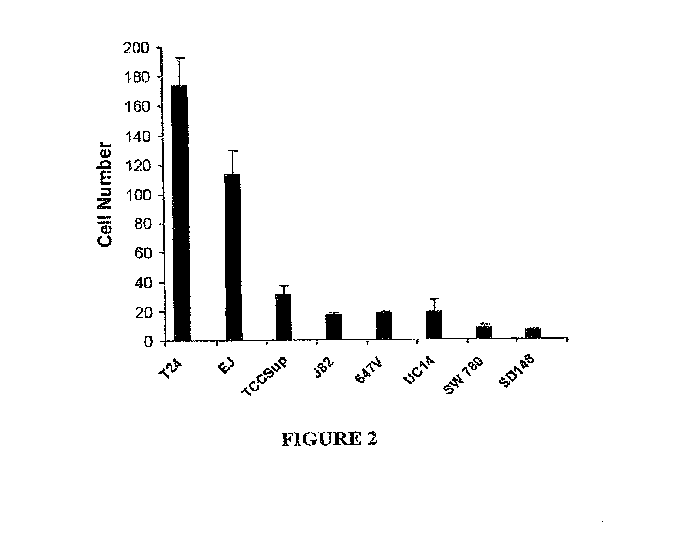

To evaluate the association of N-Cadherin with invasive potential, all cell lines were evaluated in Boyden chamber assays or in an in vitro reconstitution model described previously. The latter was used to mimic the in vivo relationship of epithelium and stroma. As shown in Figure 2, the N-cadherin positive cell lines all exhibited varying degrees of invasive behavior, while the N-cadherin null cells were minimally invasive. EJ was highly invasive in both Boyden chamber assays and the in vitro reconstitution model, while SW 780 did not invade in either assay, suggesting a good degree of correlation between the two tests. Another cell line, 647V, was as invasive as J82. Interestingly, 647V does not express N-Cadherin, but does express high levels of activated Akt. These findings support a link between invasion and Akt phosphorylation, even in the absence of N-Cadherin.

### Example 3. P-AKT pathway activation and inhibition depend on N-cadherin or PEGFR expression in invasive human bladder cell lines.

The PI3 kinase-Akt pathway is central to tumour progression and metastasis in human cancer and is believed to play a critical role in bladder cancer invasion. However, little is known about the upstream signals that activate Akt in bladder cancer. The N-cadherin and epidermal growth factor receptor (EGFR) signalling pathways were investigated in order to evaluate their involvement in activating Akt in several invasive human bladder cell lines. The molecular and functional effects of N-cadherin and EGFR inhibition in these cell lines was also investigated.

A panel of invasive and noninvasive bladder cancer cell lines were screened for activated EGFR, N-cadherin, E-cadherin, activated Akt and PTEN expression by Western Blot. Cells were evaluated with and without the EGFR antagonist Iressa, the N-cadherin blocking antibody GC-4, and the PI3K inhibitor LY294002. The invasive behaviour of each cell line was also evaluated in the presence or absence of the above molecular inhibitors.

There was an inverse relationship between N-Cadherin and E-Cadherin expression. The T24, J82 and TCCsup cell lines were strongly N-Cadherin positive and E-Cadherin negative. With a single exception (eg. the EJ cell line), there was also an inverse relationship between N-cadherin and activated EGFR (pEGFR) expression. All N-cadherin positive cell lines (T24-EJ-J82-TCCSup) strongly expressed activated Akt (pAkt), while only a single pEGFR positive cell line (eg.647V) did. In an in *vitro* invasion assay, only N-cadherin and pEGFR positive cell lines with strong pAkt expression were invasive. GC4 and Iressa downregulated pAkt expression in all cell lines. Likewise, GC-4 and Iressa significantly decreased invasion of T24-EJ-J82 and 647V, respectively. LY294002 was as efficient as GC-4 or Iressa in blocking the invasiveness of pAkt positive cell lines, suggesting that pAkt is critical for N-Cadherin and pEGFR-mediated invasion. Intriguingly, N-Cadherin blockade with GC-4 not only reduced Akt activation, but also restored E-Cadherin expression. Similarly, Iressa treatment increased E-Cadherin expression in all pEGFR positive cell lines (647V-SD148-RT112), even those that were only weakly pAkt positive and noninvasive. In contrast, Ly294002 treatment did not restore E-Cadherin expression.

### Example 4. N-cadherin and pEGFR represent alternative Akt activating pathways required for invasive bladder cancer behavior.

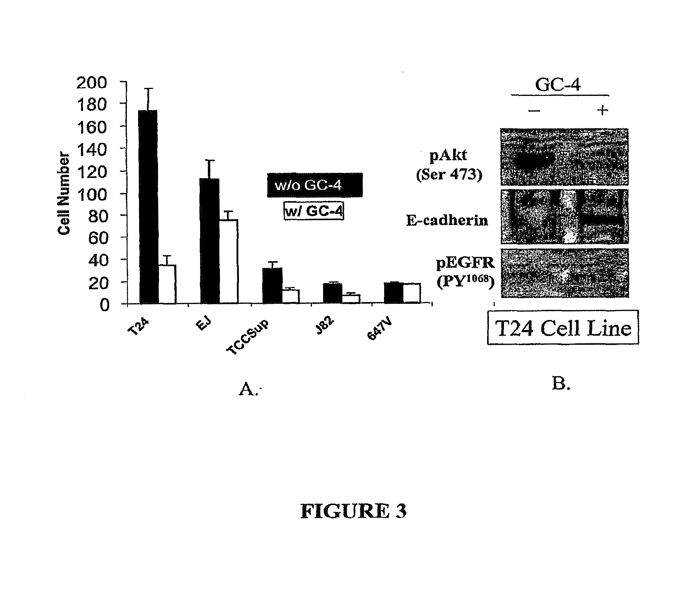

As shown in Figure 3A, treatment ofN-Cadherin positive cell lines TCC, EJ, T24 and J82 significantly reduced invasion, whereas it had no effect on the invasive N-Cadherin null cell line 647V. We also assessed EJ invasiveness with and without GC-4 in the in vitro reconstitution model and saw a 50% reduction in invasion into the rat stroma on which the cells were cultured (data not shown). These results suggest that N-Cadherin blockade can specifically inhibit invasion of N-Cadherin positive cell lines.

In order to determine if neutralization of endogenous N-Cadherin inhibited Akt activation, Western blotting of treated cells was performed. As shown in Figure 3B, GC-4 treatment significantly reduced Akt phosphorylation in the T24 cell line. Similar results were seen in the other N-Cadherin cell lines. We also asked whether N-Cadherin neutralization might reverse the cadherin switch characteristic of EMT. Figure 3B shows that GC-4 treatment restores E-Cadherin expression to the previously E-Cadherin negative T24 cell line. These results suggest that N-cadherin blockade may inhibit invasion by inactivating Akt and restoring E-Cadherin expression.

N-Cadherin and pEGFR blockade are sufficient to block invasion and appear to do so by downregulating Akt. The ability of the PI3 kinase inhibitor to block invasion in these cell lines highlights the critical role of Akt in this process and indicates that N-Cadherin and pEGFR activate Akt via the PI3 kinase cascade. GC-4 and Iressa, but not Ly294002, can restore or increase E-Cadherin expression, suggesting that E-Cadherin is not regulated directly by Akt.

In parallel experiments, we compared gene expression in paired hormone dependent and independent prostate cancers xenografts. We found that N-Cadherin is consistently upregulated in hormone refractory xenografts and autopsy cases of men who died from prostate cancer, suggesting that prostate cancers may also progress by undergoing an EMT. Recently, we have shown that *de novo* N Cadherin expression can confer invasive and androgen independent growth to an androgen responsive prostate cancer cell lines. We have also generated a series of new monoclonal antibodies against N-Cadherin.

Accordingly, N-cadherin expression can contribute to prostate and bladder cancer invasion and metastasis as well as the progression of prostate caner to hormone refractory disease. N-Cadherin can be targeted therapeutically both alone and in combination with other small molecule inhibitors of mTOR and EGFR. Targeting N-Cadherin can help prevent or control invasive and metastatic prostate cancer.

### Example 5. Activated Akt is only partially responsible for bladder cancer invasion.

In order to understand the role of AKT in invasion in bladder cancer cells, SW780, an N-Cadherin and pAKT negative cell line (Figure 1A), was infected with lentivirus containing activated AKT. Lentivirus-containing GFP was used as a negative control. The SW780 cells were examined for invasiveness using a Boyden chamber assay and, as expected, activated AKT cells were much more invasive (Figure 4A). To confirm the role of endogenous pAKT, T24 cells (known to have high pAKT and N-Cadherin) were then treated with the PI3 Kinase inhibitor LY 294002. pAkt expression was strongly inhibited by 10 ug of LY 294002 (Figure 4B). As shown in Figure 4C, LY 294002 reduced bladder cancer invasion, but to a lesser degree than the N-Cadherin antibody GC-4, even though both GC-4 and LY inhibited Akt activation equally on Western blot. These results suggest that pAkt only partially contributes to the invasion of N-cadherin positive bladder cancer cells. LY 294002 treatments did not result in E-Cadherin re-expression, as was seen after exposure of cells to GC-4 (data not shown). Also, forced pAkt expression in SW780 cells did not reduce E-Cadherin expression (figure 4A). The absence of E-cadherin expression following PI3K blockade and the failure of pAkt expression to diminish E-Cadherin suggests that N-Cadherin-mediated regulation of E-Cadherin is not Akt dependent It further suggests that N-Cadherin's contribution to invasion is caused both by an increase in pAkt and a decrease in E-Cadherin expression.

### Example 6. N-Cadherin expression is inversely related to E-Cadherin in clinical cases of superficial and invasive bladder cancer:

In order to extend our in vitro observations to human cases of bladder cancer, we first surveyed a panel of 17 freshly obtained superficial and invasive cancers for N- and E-Cadherin expression by Western blot to determine if N-Cadherin is indeed expressed by human bladder tumors. All but one tumor was from a patient with T1 or invasive cancer and virtually all were high grade. As shown in Figure 5A, fourteen of 17 (66%) tumors expressed some degree of N-Cadherin protein. Seven of these fourteen expressed low levels of E-Cadherin, while another seven had no detectable E-Cadherin. Overall, there was a strong inverse correlation between N- and E-Cadherin expression. Tumors with the strongest expression of N-Cadherin tended to have absent E-Cadherin, while tumors that were strongly E-Cadherin positive were N-Cadherin negative. Long-term follow-up for this panel of patients was not available, but the data demonstrate that N-Cadherin is commonly expressed among high grade superficial and invasive bladder cancers at levels that correspond to those seen in bladder cancer cell lines. In addition, the data support an inverse relationship between N-Cadherin and E-Cadherin expression, although it is noteworthy that many tumors do co-express both genes.

### Example 7. N-Cadherin expression is associated with poor prognosis in patients with invasive bladder cancer

The previous data confirm that N-Cadherin protein is expressed in human bladder cancer. In order to determine if N-Cadherin expression and evidence of EMT has prognostic significance in bladder cancer, we compared survival among a group of patients who underwent radical cystectomy for invasive bladder cancer based on their expression of N-Cadherin. Because N-Cadherin antibody stains paraffin-embedded tissue sections poorly (data not shown), we scored N-Cadherin expression based on RNA expression as described in Methods and Materials (Figure 5B). In short, we obtained data on N-Cadherin RNA expression from Affymetrix chips and scored tumors as positive or negative based on expression relative to the mean. RT-PCR was used to confirm the validity of this methodology on a subset of 15 patients for whom RNA was available. The correlation of RT-PCR and the information derived from the Affymetrix chips was highly significant, confirming the validity of this approach. As shown in Figure 5B, patients with N-Cadherin positive tumors had a significantly shorter overall survival compared to patients whose tumors did not express N-cadherin (p - 0.0064).

Next, we stratified patients based on both N-Cadherin and E-Cadherin expression. E-Cadherin expression was scored and validated similarly to N-cadherin. Four groups of patients were identified: those expressing N-Cadherin alone, those expressing both E-Cadherin and N-Cadherin, those expressing neither, and those expressing E-Cadherin alone. Patients expressing both N- and E-Cadherin and those expressing neither had similar survival curves and were plotted together. As shown in Figure 6, overall survival stratified clearly into three groups, with N-Cadherin positive tumors having the worst prognosis and E-Cadherin positive tumors the best. The mixed tumors had an intermediate prognosis. These data show that N-Cadherin and E-Cadherin expression combine to determine prognosis among patients with invasive bladder cancer. The presence of N-Cadherin confers a worse prognosis and provides additional information to that obtained by the use of E-cadherin alone. In fact, N-Cadherin is a stronger independent marker of prognosis than E-Cadherin.

### Example 8. N-Cadherin is upregulated in hormone refractory prostate cancer xenografts, cell lines and patient tumors.

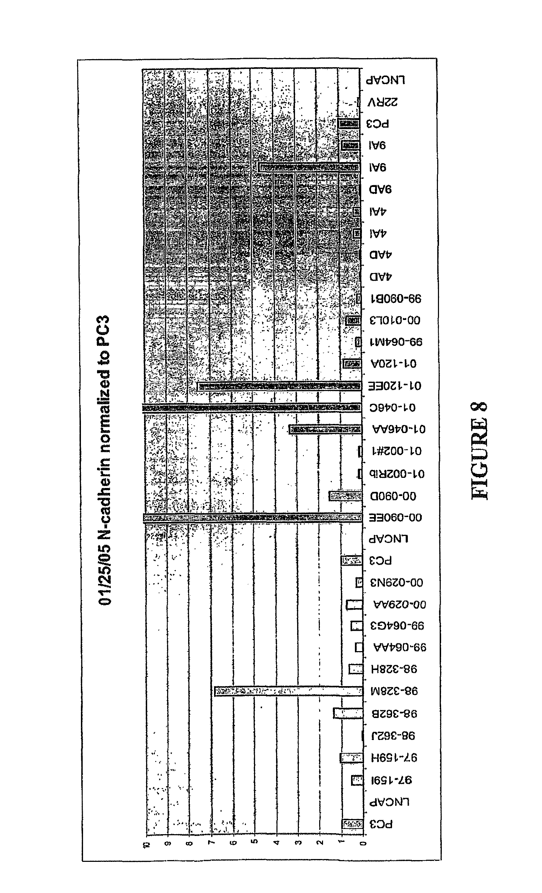

Over the past few years, our laboratory has compared gene expression in paired hormone sensitive and independent LAPC-4 and LAPC-9 prostate cancer xenografts. Using these models, we previously cloned Reg*IV, a secreted protein that is upregulated in 60% of hormone refractory tumors. Another gene consistently found to be upregulated in LAPC-4 and 9 AI (androgen independent) tumors was N-Cadherin. Real-time PCR (Figure 8) and Western blot analysis (Figure 7) confirm that N-Cadherin is increased consistently in independently derived AI clones. N-Cadherin is also expressed in the AI cell lines 22RV1 and PC3, but not in the androgen dependent cell line LNCaP (not shown). These data are consistent with previous reports by Tomita et al. and others that >60% of hormone refractory prostate cancer metastases are N-Cadherin positive. High grade primary tumors (Gleason 8-10) also expressed N-Cadherin (~40%), while low grade tumors were rarely positive. We also compared N Cadherin expression in a cohort of autopsy derived prostate cancer metastases. A majority of these androgen independent tumors express levels of N-Cadherin comparable to PC3, a cell line with levels similar to the invasive bladder cancer lines shown above. These results suggest that EMT and N-Cadherin expression may also be important in prostate cancer, either by promoting metastasis or progression to androgen independence.

### Example 9. N-Cadherin Expression Promotes Invasive and Androgen Independent Growth.

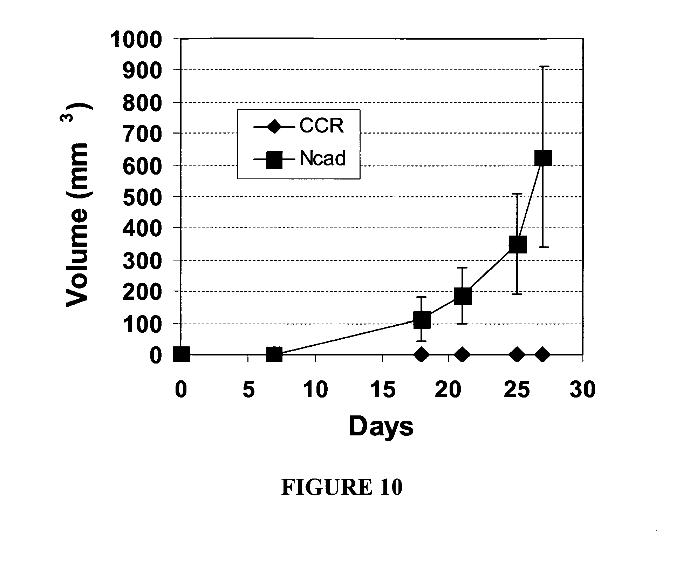

In order to determine ifN-cadherin expression could result in either invasive, metastatic or androgen independent growth, we stably infected the androgen dependent LNCaP cell line with lentivirus containing an N-Cadherin construct. LNCaP cells transduced with N-Cadherin had detectable levels of N-Cadherin (Figure 9), a markedly altered morphology compared to control infected cells (not shown), and invaded matrigel chambers significantly greater than controls (Figure 9). LNCaP-N Cadherin cells survived in charcoal stripped serum, suggesting acquisition of androgen independence. Most strikingly, these cells formed tumors rapidly in castrate mice, while control cells did not form tumors, suggesting both increased tumorigenicity and androgen independence (Figure 10). Androgen receptor levels in early passages were unchanged, suggesting that this effect is independent of the AR. Finally, N-Cadherin neutralization reduced invasion of these cells, consitent with the previous results in bladder cancer. These results provide the first existing evidence known to us that N-Cadherin expression can (1) cause and EMT in prostate cancer cells (2) confer invasive behavior to prostate cancer cells, and (3) confer androgen independent growth. Finally, N-Cadherin neutralization inhibited invasion, suggesting that N-cadherin could be a therapeutic target in prostate cancer cells with evidence of EMT. N-cadherin overexpression was also found to cause androgen-independent cell cycle activation and growth in LNCaP cells and to abolish androgen sensitivity in the growth of LNCaP cells.

These findings demonstrate that an EMT characterized by induction of N-Cadherin can increase invasiveness of bladder cancer, which translates clinically into an increased risk of metastasis and death. This biologic effect is regulated in part by activation of Akt and loss of E-Cadherin and can be targeted therapeutically by antibodies against N-Cadherin. In addition to bladder cancer, our preliminary results suggest that N-Cadherin is upregulated in hormone-refractory prostate cancers. This is consistent with other reports of N-Cadherin expression in advanced prostate cancer, although to date no-one has explored an association with prognosis. Likewise, no-one has explored the concept of targeting N-Cadherin in prostate cancer therapeutically or the biological contribution of N-Cadherin to hormone refractory prostate cancer.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

## Claims

1. An N-cadherin siRNA or an anti-N-cadherin antibody for inhibiting the growth of a cancer that overexpresses N-cadherin, wherein the cancer is likely to become hormone. independent, refractory to treatment, or recurrent.

2. The N-cadherin siRNA or anti-N-cadherin antibody of claim 1, wherein the cancer is urogenital cancer.

3. The N-cadherin siRNA or anti-N-cadherin antibody of claim 1 or 2, wherein the cancer is prostate cancer or bladder cancer.

4. The N-cadherin siRNA or anti-N-cadherin antibody of any of claims 1-3, wherein the siRNA or the antibody is specific for the first extracellular domain, portions of the first and second extracellular domains, or the fourth extracellular domain.

5. The N-cadherin siRNA or anti-N-cadherin antibody of any of claims 1-4, wherein the antibody is a monoclonal antibody.

6. The N-cadherin siRNA or anti-N-cadherin antibody of any of claims 1-5, wherein the antibody is conjugated to an effector moiety.

7. The N-cadherin siRNA or an anti-N-cadherin antibody of claim 6, wherein the effector moiety is a cytotoxic agent.

8. A method of targeting patients for more aggressive or alternative cancer therapy or increased surveillance for a cancer recurrence based upon whether there is an increased likelihood of the cancer becoming hormone independent, refractory to treatment, or recurrent, the method comprising the steps of:
(a) determining the presence or amount of N-cadherin protein or mRNA in a test tissue sample from a test tissue individual; and
(b) comparing the result obtained in step (a) with a control tissue sample from an individual known to be negative for the cancer;
wherein an overexpression of the N-cadherin protein or mRNA in the test tissue sample indicates that the test individual has cancer which is likely to become hormone independent, refractory to treatment, or recurrent.

9. The method of claim 8, wherein the test tissue sample is contacted with an antibody that specifically binds to N-cadherin protein.

10. The method of claim 8 or 9, wherein the test tissue sample is contacted with a primer set of a first oligonucleotide and a second oligonucleotide that each specifically hybridize to N-cadherin mRNA to amplify N-cadherin mRNA.

11. The method of any of claims 8-10, wherein the cancer is urogenital cancer.

12. The method of any of claims 8-11, wherein the cancer is prostate cancer or bladder cancer.

13. The method of any of claims 8-12, wherein the overexpression is by at least four-fold over the control tissue sample.

14. The method of any of claims 8-13, wherein the test tissue sample is a biopsy sample.
